# EUROPEAN PATENT APPLICATION

(11) **EP 4 620 483 A1**
(43) Date of publication of application: **24.09.2025**
(21) Application number: 23890840.4
(22) Date of filing: 16.11.2023
(51) Int. Cl.: A61K 39/395, C07K 16/28, A61P 19/08, A61P 29/00

(54) **PHARMACEUTICAL COMPOSITION COMPRISING ANTI-RANKL-NGF BISPECIFIC ANTIBODIES**

(30) Priority: 16.11.2022 CN 202211438239
(71) Applicant: Suzhou Suncadia Biopharmaceuticals Co., Ltd., Suzhou, Jiangsu 215126 (CN); Shanghai Hengrui Pharmaceutical Co., Ltd., Shanghai 200245 (CN); Jiangsu Hengrui Pharmaceuticals Co., Ltd., Lianyungang, Jiangsu 222047 (CN)
(72) Inventor: YANG, Zhen, Suzhou, Jiangsu 215126 (CN); YANG, Xiqin, Suzhou, Jiangsu 215126 (CN); GE, Lingxiao, Suzhou, Jiangsu 215126 (CN); WANG, Hongwei, Suzhou, Jiangsu 215126 (CN)
(74) Representative: Potter Clarkson
(86) International application number: PCT/CN2023/131913
(87) International publication number: WO 2024/104409

(57) **Abstract**

The present disclosure relates to a pharmaceutical composition comprising an anti-RANKL-NGF bispecific antibodies. Specifically, the pharmaceutical composition of the present disclosure comprises anti-RANKL-NGF bispecific antibodies and a buffer.

## Description

The present application claims priority to Chinese Patent Application No. 202211438239.8 (filed on November 16, 2022).

### TECHNICAL FIELD

The present disclosure relates to the field of pharmaceutical formulations, in particular to a pharmaceutical composition comprising an anti-RANKL-NGF bispecific antibody and use thereof as a medicament.

### BACKGROUND

The statements herein merely provide background information related to the present disclosure and may not necessarily constitute the prior art.

Bone metastasis is a common accompanying symptom in later stages of many cancers. The pathogenesis of bone metastasis: Primary tumor cells fall off and enter the blood circulation system, forming circulating tumor cells, and the circulating tumor cells are transferred to a bone part, form transmissible tumor cells and lie dormant in a bone microenvironment. This process may continue for years until the surviving tumor cells gradually adapt to the bone microenvironment. The tumor cells are activated, transitioning from the dormant state to a proliferative state (Mantyh, P.W, Curr Opin Support Palliat Care, 2014. 8(2): p83-90). The proliferation of the tumor cells affects the bone microenvironment and promotes osteoclast formation, which leads to accelerated bone resorption and release of various cytokines, further promoting tumor cell proliferation. Hence, a vicious cycle is formed (Quayle, L., Current Cancer Drug Targets, 2015. 15(6): p469-480). The constant proliferation of tumor cells leads to the formation of multiple metastatic sites, which causes various problems such as bone injury and pain in patients (Gartland, A., Journal of Bone Oncology, 2016. 5(3): p100-103).

According to statistics, common cancers involving bone metastasis include myeloma, renal cancer, melanoma, bladder cancer, thyroid cancer, lung cancer, breast cancer, and prostate cancer (Clezardin, P., Joint Bone Spine, 2017. 84(6): p677-684. Fidler, M.M., Fidler, Scandinavian Journal of Public Health, 2018. 46(1): p27-36).

In normal bone tissue, osteoblasts and osteoclasts are in a relatively balanced state to maintain the normal growth and development of bones. However, the presence of cancer cells will break the balance. Cancer cells in bone microenvironments release a series of cytokines to stimulate the secretion of a large amount of receptor activator of nuclear factor-κB ligand (RANKL) from osteoblasts and osteocytes. The interaction of RANKL with the receptor RANK promotes osteoclast formation, leading to increased bone resorption. Osteolysis caused by bone resorption causes the release of other growth factors to promote cancer cell proliferation. Hence, a cycle that favors the metastasis of cancer cells is formed (Body, J.J., Expert Rev Anticancer Ther, 2012. 12(3): p307-322).

NGF (nerve growth factor) is a nerve growth factor. The NGF signaling pathway mediates the growth and development of the nervous system and the transmission of pain signals. At present, there are two known receptors for NGF on the cell surface: the high-affinity receptor TrKA and the low-affinity receptor p75NTR. The interaction of NGF with TrkA activates the Ras and PI3K pathways simultaneously, promoting cell survival and nerve growth. The interaction of NGF with p75NTR promotes apoptosis. Moreover, the PI3K pathway also activates the phosphorylation of TRPV1, causing ion channels to be activated to generate action potentials and transmit signals from pain neurons (Kumar, V. and Mahal B. A., Journal of Pain Research, 2012. 5: p279-287).

Existing data have shown that monoclonal antibodies targeting NGF and RANKL have certain therapeutic effects on bone injury and pain caused by bone metastasis (Body JJ. Expert Rev Anticancer Ther. 2012. 12(3): p307-322. Sopata M., et al. 2015. 156(9): p1703-1713).

### SUMMARY

The present disclosure provides a pharmaceutical composition comprising an anti-RANKL-NGF bispecific antibody, and the composition has therapeutic activity. In addition, the composition also has the advantages of good stability and the like.

In some embodiments, the present disclosure provides a pharmaceutical composition comprising an anti-RANKL-NGF bispecific antibody and a buffer, wherein the anti-RANKL-NGF bispecific antibody comprises a first antigen-binding domain that specifically binds to RANKL and a second antigen-binding domain that specifically binds to NGF, and the buffer is an acetate buffer, a histidine buffer, or a phosphate buffer.

In some embodiments, the buffer is an acetic acid-sodium acetate buffer, a histidine-histidine hydrochloride buffer, or a citric acid-disodium hydrogen phosphate buffer.

In some embodiments, the buffer is an acetic acid-sodium acetate buffer or a histidine-histidine hydrochloride buffer.

In some specific embodiments, the buffer is an acetic acid-sodium acetate buffer.

In some embodiments, provided is the pharmaceutical composition according to any one of the above, wherein the pharmaceutical composition has a pH of 4.2 to 7.8. In some embodiments, the pharmaceutical composition has a pH of 4.2 to 7.0. In some embodiments, the pharmaceutical composition has a pH of 4.2 to 5.4. In some embodiments, the pharmaceutical composition has a pH of 4.6 to 5.4. In some embodiments, the pharmaceutical composition has a pH of about 4.6. In some embodiments, the pharmaceutical composition has a pH of about 4.8. In some embodiments, the pharmaceutical composition has a pH of about 5.0. In some embodiments, the pharmaceutical composition has a pH of about 5.2. In some embodiments, the pharmaceutical composition has a pH of about 5.4. When a point value is referred to in the present disclosure, it should be understood that the point value includes an error range. This error range is due to factors such as laboratory environment, personnel operations, instruments, methodology, measurement errors, and the like. Taking the pH as an example, when the measurement is about 5.0, it should be understood that an error range is included. As an example, when the formulation is measured using an industrial pH meter, "about 5.0" represents 5.0±0.2 (i.e., pH of 4.8 to 5.2).

In some embodiments, the pharmaceutical composition has a pH of 4.6-6.6. In some embodiments, the pharmaceutical composition has a pH of 4.6-5.8. In some embodiments, the pharmaceutical composition has a pH of 5.0-5.8.

In some embodiments, the pharmaceutical composition has a pH of 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, or 7.8, or any range between these point values. In some embodiments, the pharmaceutical composition has a pH of 4.6. In some embodiments, the pharmaceutical composition has a pH of 4.8. In some embodiments, the pharmaceutical composition has a pH of 5.0. In some embodiments, the pharmaceutical composition has a pH of 5.2. In some embodiments, the pharmaceutical composition has a pH of 5.4.

Generally, the pH of the pharmaceutical composition obtained by replacing the buffer is almost consistent with the pH of the buffer. Also, it is well known to those skilled in the art that in the process of pharmaceutical formulation, there may sometimes be a pH drift, but the pH drift of the pharmaceutical formulation is generally small (e.g., within a range of ±0.3). In some embodiments, the pH drift of the pharmaceutical formulation is within a range of ±0.2. In some embodiments, the pH drift of the pharmaceutical formulation is within a range of ±0.1.

In some embodiments, provided is the pharmaceutical composition according to any one of the above, wherein the anti-RANKL-NGF bispecific antibody is at a concentration of 1 mg/mL to 150 mg/mL. In some embodiments, the anti-RANKL-NGF bispecific antibody is at a concentration of 1 mg/mL to 100 mg/mL. In some embodiments, the anti-RANKL-NGF bispecific antibody is at a concentration of 10 mg/mL to 80 mg/mL. In some embodiments, the anti-RANKL-NGF bispecific antibody is at a concentration of 20 mg/mL to 80 mg/mL. In some embodiments, the anti-RANKL-NGF bispecific antibody is at a concentration of 20 mg/mL to 77 mg/mL. In some embodiments, the anti-RANKL-NGF bispecific antibody is at a concentration of 20 mg/mL to 70 mg/mL. In some embodiments, the anti-RANKL-NGF bispecific antibody is at a concentration of 50 mg/mL to 77 mg/mL. In some embodiments, the anti-RANKL-NGF bispecific antibody is at a concentration of 56 mg/mL to 84 mg/mL. In some embodiments, the anti-RANKL-NGF bispecific antibody is at a concentration of 63 mg/mL to 77 mg/mL. In some embodiments, the anti-RANKL-NGF bispecific antibody is at a concentration of about 77 mg/mL. In some embodiments, the anti-RANKL-NGF bispecific antibody is at a concentration of about 70 mg/mL. In some embodiments, the anti-RANKL-NGF bispecific antibody is at a concentration of about 50 mg/mL. In some embodiments, the anti-RANKL-NGF bispecific antibody is at a concentration of about 20 mg/mL. In some embodiments, the anti-RANKL-NGF bispecific antibody is at a concentration of 1 mg/mL, 5 mg/mL, 10 mg/mL, 20 mg/mL, 30 mg/mL, 40 mg/mL, 45 mg/mL, 50 mg/mL, 55 mg/mL, 56 mg/mL, 60 mg/mL, 63 mg/mL, 65 mg/mL, 70 mg/mL, 75 mg/mL, 77 mg/mL, 80 mg/mL, 84 mg/mL, 90 mg/mL, 100 mg/mL, 110 mg/mL, 120 mg/mL, 130 mg/mL, 140 mg/mL, or 150 mg/mL, or any range between these point values. In some embodiments, the anti-RANKL-NGF bispecific antibody is at a concentration of 77 mg/mL. In some embodiments, the anti-RANKL-NGF bispecific antibody is at a concentration of 70 mg/mL. In some embodiments, the anti-RANKL-NGF bispecific antibody is at a concentration of 50 mg/mL. In some embodiments, the anti-RANKL-NGF bispecific antibody is at a concentration of 20 mg/mL.

In some embodiments, provided is the pharmaceutical composition according to any one of the above, wherein the pharmaceutical composition comprises a surfactant. In some embodiments, the surfactant is a nonionic surfactant. In some embodiments, the surfactant is selected from the group consisting of poloxamer (e.g., poloxamer 188), polysorbate (e.g., polysorbate 20 or polysorbate 80), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymers of ethylene and propylene glycol, and the like. In some embodiments, the surfactant is polysorbate or poloxamer. In some embodiments, the surfactant is polysorbate 80, polysorbate 20, or poloxamer 188. In some embodiments, the surfactant is polysorbate 80 or polysorbate 20. In some embodiments, the surfactant is polysorbate 80.

In some embodiments, provided is the pharmaceutical composition according to any one of the above, wherein the surfactant is at a concentration of 0.01 mg/mL to 1.0 mg/mL. In some embodiments, the surfactant is at a concentration of 0.01 mg/mL to 0.8 mg/mL. In some embodiments, the surfactant is at a concentration of 0.01 mg/mL to 0.6 mg/mL. In some embodiments, the surfactant is at a concentration of 0.01 mg/mL to 0.4 mg/mL. In some embodiments, the surfactant is at a concentration of 0.01 mg/mL to 0.2 mg/mL. In some embodiments, the surfactant is at a concentration of 0.1 mg/mL to 0.4 mg/mL. In some embodiments, the surfactant is at a concentration of 0.1 mg/mL to 0.2 mg/mL. In some embodiments, the surfactant is at a concentration of 0.05 mg/mL to 0.15 mg/mL. In some embodiments, the surfactant is at a concentration of 0.08 mg/mL to 0.12 mg/mL. In some embodiments, the surfactant is at a concentration of 0.09 mg/mL to 0.11 mg/mL. In some embodiments, the surfactant is at a concentration of about 0.4 mg/mL. In some embodiments, the surfactant is at a concentration of about 0.2 mg/mL. In some embodiments, the surfactant is at a concentration of about 0.1 mg/mL. In some embodiments, the surfactant is at a concentration of 0.01 mg/mL, 0.05 mg/mL, 0.08 mg/mL, 0.09 mg/mL, 0.1 mg/mL, 0.11 mg/mL, 0.12 mg/mL, 0.15 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1.0 mg/mL, or any range between these point values. In some embodiments, the surfactant is at a concentration of 0.4 mg/mL. In some embodiments, the surfactant is at a concentration of 0.2 mg/mL. In some embodiments, the surfactant is at a concentration of 0.1 mg/mL.

In some embodiments, the surfactant is about 0.1 mg/mL polysorbate 80. In some embodiments, the surfactant is 0.1 mg/mL polysorbate 80.

In some embodiments, provided is the pharmaceutical composition according to any one of the above, which comprises a stabilizer. In some embodiments, the stabilizer is a sugar (including monosaccharide, disaccharide, trisaccharide, polysaccharide, sugar alcohol, reducing sugar, non-reducing sugar, and the like), an amino acid (including proline, arginine, glycine, cysteine, histidine, and the like), or a salt (sodium chloride, potassium chloride, calcium chloride, and the like). In some embodiments, the stabilizer is selected from one or more of the group consisting of proline, sucrose, trehalose, sorbitol, arginine, glycine, and sodium chloride. In some embodiments, the stabilizer is proline, sucrose, or sodium chloride. In some embodiments, the stabilizer is an amino acid. In some embodiments, the stabilizer is proline.

In some embodiments, provided is the pharmaceutical composition according to any one of the above, wherein the stabilizer is at a concentration of 1 mM to 300 mM. In some embodiments, the stabilizer is at a concentration of 25 mM to 290 mM. In some embodiments, the stabilizer is at a concentration of 25 mM to 250 mM. In some embodiments, the stabilizer is at a concentration of 210 mM to 270 mM. In some embodiments, the stabilizer is at a concentration of 216 mM to 264 mM. In some embodiments, the stabilizer is at a concentration of 228 mM to 252 mM. In some embodiments, the stabilizer is at a concentration of about 240 mM. In some embodiments, the stabilizer is at a concentration of 1 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, 200 mM, 210 mM, 216 mM, 220 mM, 228 mM, 230 mM, 235 mM, 240 mM, 245 mM, 250 mM, 252 mM, 260 mM, 264 mM, 270 mM, 280 mM, 290 mM, or 300 mM, or any range between these point values. In some embodiments, the stabilizer is at a concentration of 240 mM. In some embodiments, the stabilizer is about 240 mM proline. In some embodiments, the stabilizer is 240 mM proline.

In some embodiments, the stabilizer is a sugar, and the sugar is selected from the group consisting of glucose, sucrose, trehalose, lactose, fructose, maltose, dextran, glycerin, erythritol, glycerol, arabitol, xylitol, sorbitol (also known as sorbol), mannitol, melibiose, melezitose, raffinose, mannotriose, stachyose, maltose, lactulose, maltulose, maltitol, lactitol, and iso-maltulose. In some embodiments, the stabilizer is selected from one or more of the group consisting of sucrose, trehalose, sorbitol, arginine, glycine, and sodium chloride. In some embodiments, the stabilizer is a non-reducing disaccharide. In some embodiments, the stabilizer is trehalose or sucrose. In some embodiments, the stabilizer is sucrose.

In some embodiments, the stabilizer is 10 mg/mL to 100 mg/mL sucrose. In some embodiments, the stabilizer is 30 mg/mL to 80 mg/mL sucrose. In some embodiments, the stabilizer is 50 mg/mL to 80 mg/mL sucrose. In some embodiments, the stabilizer is 70 mg/mL to 80 mg/mL sucrose. In some embodiments, the stabilizer is 60 mg/mL to 90 mg/mL sucrose. In some embodiments, the stabilizer is 67.5 mg/mL to 82.5 mg/mL sucrose. In some embodiments, the stabilizer is about 75 mg/mL sucrose. In some embodiments, non-limiting examples of the concentration of the stabilizer include 10 mg/mL, 20 mg/mL, 30 mg/mL, 40 mg/mL, 50 mg/mL, 60 mg/mL, 65 mg/mL, 67.5 mg/mL, 70 mg/mL, 75 mg/mL, 80 mg/mL, 82.5 mg/mL, 85 mg/mL, 90 mg/mL, 95 mg/mL, 100 mg/mL, and any range between these point values. In some embodiments, the stabilizer is 75 mg/mL sucrose.

In some embodiments, the stabilizer is a salt. In some embodiments, the stabilizer is sodium chloride. In some embodiments, the stabilizer is 0.64% (w/v) to 0.96% (w/v) sodium chloride. In some embodiments, the stabilizer is 0.72% (w/v) to 0.88% (w/v) sodium chloride. In some embodiments, the stabilizer is about 0.8% (w/v) sodium chloride. In some embodiments, the stabilizer is 0.8% (w/v) sodium chloride.

In some embodiments, provided is the pharmaceutical composition according to any one of the above, wherein the buffer is at a concentration of 5 mM to 100 mM. In some embodiments, the buffer is at a concentration of 10 mM to 50 mM. In some embodiments, the buffer is at a concentration of 10 mM to 30 mM. In some embodiments, the buffer is at a concentration of 10 mM to 20 mM. In some embodiments, the buffer is at a concentration of 16 mM to 24 mM. In some embodiments, the buffer is at a concentration of 18 mM to 22 mM. In some embodiments, the buffer is at a concentration of about 20 mM. In some embodiments, the buffer is at a concentration of about 10 mM. In some embodiments, the buffer is at a concentration of 5 mM, 10 mM, 15 mM, 16 mM, 18 mM, 20 mM, 22 mM, 24 mM, 25 mM, 30 mM, 40 mM, 50 mM, 60 mM, 70 mM, 80 mM, 90 mM, or 100 mM, and any range between these point values. In some embodiments, the buffer is at a concentration of 20 mM. In some embodiments, the buffer is at a concentration of 10 mM.

In some embodiments, the buffer is about 10 mM acetic acid-sodium acetate buffer. In some embodiments, the buffer is about 20 mM acetic acid-sodium acetate buffer. In some embodiments, the buffer is 10 mM acetic acid-sodium acetate buffer. In some embodiments, the buffer is 20 mM acetic acid-sodium acetate buffer. In some embodiments, the buffer is 10 mM histidine-histidine hydrochloride buffer. In some embodiments, the buffer is 10 mM citric acid-disodium hydrogen phosphate buffer.

In some embodiments, provided is the pharmaceutical composition according to any one of the above, wherein the anti-RANKL-NGF bispecific antibody comprises: at least one first antigen-binding domain that specifically binds to RANKL and at least one second antigen-binding domain that specifically binds to NGF.

In some embodiments, provided is the pharmaceutical composition according to any one of the above, wherein the anti-RANKL-NGF bispecific antibody comprises: two first antigen-binding domains that specifically bind to RANKL and two second antigen-binding domains that specifically bind to NGF.

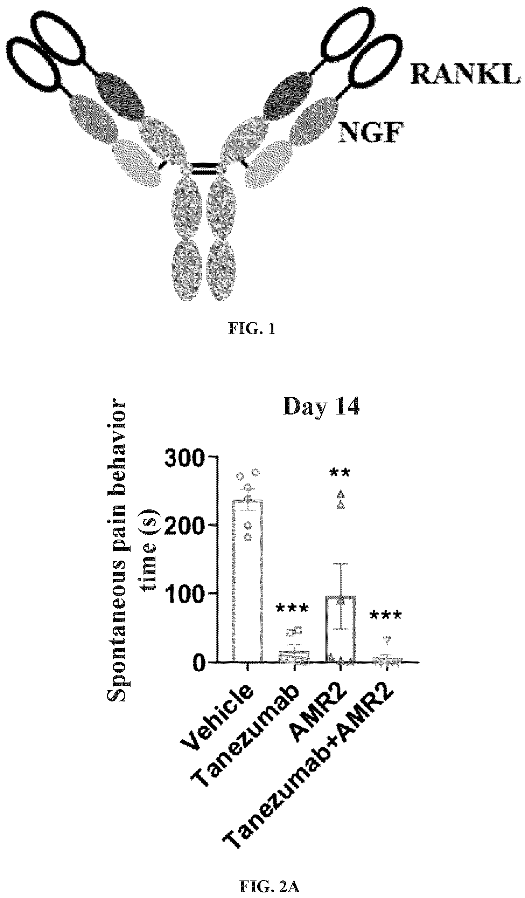

In some embodiments, provided is the pharmaceutical composition according to any one of the above, wherein the anti-RANKL-NGF bispecific antibody has a structure as shown in FIG. 1.

In some embodiments, provided is the pharmaceutical composition according to any one of the above, wherein in the anti-RANKL-NGF bispecific antibody, the first antigen-binding domain that specifically binds to RANKL comprises a heavy chain variable region and a light chain variable region, wherein:
the heavy chain variable region comprises: a HCDR1 comprising the amino acid sequence of SEQ ID NO: 1, a HCDR2 comprising the amino acid sequence of SEQ ID NO: 2, and a HCDR3 comprising the amino acid sequence of SEQ ID NO: 3; and
the light chain variable region comprises: a LCDR1 comprising the amino acid sequence of SEQ ID NO: 4, a LCDR2 comprising the amino acid sequence of SEQ ID NO: 5, and a LCDR3 comprising the amino acid sequence of SEQ ID NO: 6.

In some embodiments, provided is the pharmaceutical composition according to any one of the above, wherein in the anti-RANKL-NGF bispecific antibody, the first antigen-binding domain that specifically binds to RANKL comprises a heavy chain variable region and a light chain variable region, wherein the heavy chain variable region comprises the amino acid sequence of SEQ ID NO: 7; and/or the light chain variable region comprises the amino acid sequence of SEQ ID NO: 8.

In some embodiments, provided is the pharmaceutical composition according to any one of the above, wherein in the anti-RANKL-NGF bispecific antibody, the second antigen-binding domain that specifically binds to NGF comprises a heavy chain variable region and a light chain variable region, wherein:
the heavy chain variable region comprises: a HCDR1 comprising the amino acid sequence of SEQ ID NO: 15, a HCDR2 comprising the amino acid sequence of SEQ ID NO: 16, and a HCDR3 comprising the amino acid sequence of SEQ ID NO: 17; and
the light chain variable region comprises: a LCDR1 comprising the amino acid sequence of SEQ ID NO: 18, a LCDR2 comprising the amino acid sequence of SEQ ID NO: 19, and a LCDR3 comprising the amino acid sequence of SEQ ID NO: 20.

In some embodiments, provided is the pharmaceutical composition according to any one of the above, wherein in the anti-RANKL-NGF bispecific antibody, the second antigen-binding domain that specifically binds to NGF comprises a heavy chain variable region and a light chain variable region, wherein:
the heavy chain variable region comprises the amino acid sequence of SEQ ID NO: 21; and/or
the light chain variable region comprises the amino acid sequence of SEQ ID NO: 22.

In some embodiments, provided is the pharmaceutical composition according to any one of the above, wherein in the anti-RANKL-NGF bispecific antibody, the second antigen-binding domain that specifically binds to NGF comprises a heavy chain and a light chain, wherein the heavy chain comprises the amino acid sequence of SEQ ID NO: 24, and the light chain comprises the amino acid sequence of SEQ ID NO: 25.

In some embodiments, provided is the pharmaceutical composition according to any one of the above, wherein in the anti-RANKL-NGF bispecific antibody,
(i) the first antigen-binding domain that specifically binds to RANKL comprises a heavy chain variable region and a light chain variable region, wherein the heavy chain variable region comprises: a HCDR1 comprising the amino acid sequence of SEQ ID NO: 1, a HCDR2 comprising the amino acid sequence of SEQ ID NO: 2, and a HCDR3 comprising the amino acid sequence of SEQ ID NO: 3; and
   the light chain variable region comprises: a LCDR1 comprising the amino acid sequence of SEQ ID NO: 4, a LCDR2 comprising the amino acid sequence of SEQ ID NO: 5, and a LCDR3 comprising the amino acid sequence of SEQ ID NO: 6;
(ii) the second antigen-binding domain that specifically binds to NGF comprises a heavy chain variable region and a light chain variable region, wherein the heavy chain variable region comprises: a HCDR1 comprising the amino acid sequence of SEQ ID NO: 15, a HCDR2 comprising the amino acid sequence of SEQ ID NO: 16, and a HCDR3 comprising the amino acid sequence of SEQ ID NO: 17; and
the light chain variable region comprises: a LCDR1 comprising the amino acid sequence of SEQ ID NO: 18, a LCDR2 comprising the amino acid sequence of SEQ ID NO: 19, and a LCDR3 comprising the amino acid sequence of SEQ ID NO: 20.

In some embodiments, provided is the pharmaceutical composition according to any one of the above, wherein in the anti-RANKL-NGF bispecific antibody,
(i) the first antigen-binding domain that specifically binds to RANKL comprises a heavy chain variable region and a light chain variable region, wherein the heavy chain variable region comprises the amino acid sequence of SEQ ID NO: 7; and/or the light chain variable region comprises the amino acid sequence of SEQ ID NO: 8;
(ii) the second antigen-binding domain that specifically binds to NGF comprises a heavy chain variable region and a light chain variable region, wherein the heavy chain variable region comprises the amino acid sequence of SEQ ID NO: 21; and/or the light chain variable region comprises the amino acid sequence of SEQ ID NO: 22.

In some embodiments, provided is the pharmaceutical composition according to any one of the above, wherein in the anti-RANKL-NGF bispecific antibody,
(i) the first antigen-binding domain that specifically binds to RANKL comprises a heavy chain variable region and a light chain variable region, wherein the heavy chain variable region comprises: a HCDR1 comprising the amino acid sequence of SEQ ID NO: 1, a HCDR2 comprising the amino acid sequence of SEQ ID NO: 2, and a HCDR3 comprising the amino acid sequence of SEQ ID NO: 3; and
   the light chain variable region comprises: a LCDR1 comprising the amino acid sequence of SEQ ID NO: 4, a LCDR2 comprising the amino acid sequence of SEQ ID NO: 5, and a LCDR3 comprising the amino acid sequence of SEQ ID NO: 6;
(ii) the second antigen-binding domain that specifically binds to NGF comprises a heavy chain variable region and a light chain variable region, wherein the heavy chain variable region comprises: a HCDR1 comprising the amino acid sequence of SEQ ID NO: 15, a HCDR2 comprising the amino acid sequence of SEQ ID NO: 16, and a HCDR3 comprising the amino acid sequence of SEQ ID NO: 17; and
the light chain variable region comprises: a LCDR1 comprising the amino acid sequence of SEQ ID NO: 18, a LCDR2 comprising the amino acid sequence of SEQ ID NO: 19, and a LCDR3 comprising the amino acid sequence of SEQ ID NO: 20; and the anti-RANKL-NGF bispecific antibody has a structure as shown in FIG. 1.

In some embodiments, provided is the pharmaceutical composition according to any one of the above, wherein in the anti-RANKL-NGF bispecific antibody,
(i) the first antigen-binding domain that specifically binds to RANKL comprises a heavy chain variable region and a light chain variable region, wherein the heavy chain variable region comprises the amino acid sequence of SEQ ID NO: 7; and/or the light chain variable region comprises the amino acid sequence of SEQ ID NO: 8;
(ii) the second antigen-binding domain that specifically binds to NGF comprises a heavy chain variable region and a light chain variable region, wherein the heavy chain variable region comprises the amino acid sequence of SEQ ID NO: 21; and/or the light chain variable region comprises the amino acid sequence of SEQ ID NO: 22; and the anti-RANKL-NGF bispecific antibody has a structure as shown in FIG. 1.

In some embodiments, provided is the pharmaceutical composition according to any one of the above, wherein the anti-RANKL-NGF bispecific antibody comprises a first polypeptide chain and a second polypeptide chain, wherein the first polypeptide chain comprises the amino acid sequence of SEQ ID NO: 30, and the second polypeptide chain comprises the amino acid sequence of SEQ ID NO: 31.

In some embodiments, the anti-RANKL-NGF bispecific antibody comprises two first chains identical in sequence and two second chains identical in sequence, wherein the first polypeptide chain comprises the amino acid sequence of SEQ ID NO: 30, and the second polypeptide chain comprises the amino acid sequence of SEQ ID NO: 31.

In some embodiments, provided is the pharmaceutical composition according to any one of the above, which comprises the following components:
(a) 1 mg/mL to 150 mg/mL said anti-RANKL-NGF bispecific antibody,
(b) 0.01 mg/mL to 1.0 mg/mL surfactant,
(c) 1 mM to 300 mM stabilizer, and
(d) 5 mM to 100 mM buffer, the pharmaceutical composition having a pH of 4.2 to 7.0.

In some embodiments, provided is the pharmaceutical composition according to any one of the above, which comprises the following components:
(a) 1 mg/mL to 150 mg/mL said anti-RANKL-NGF bispecific antibody,
(b) 0.01 mg/mL to 1.0 mg/mL surfactant,
(c) 1 mM to 300 mM proline, and
(d) 5 mM to 100 mM buffer, the pharmaceutical composition having a pH of 4.2 to 7.0.

In some embodiments, provided is the pharmaceutical composition according to any one of the above, which comprises the following components:
(a) 10 mg/mL to 80 mg/mL said anti-RANKL-NGF bispecific antibody,
(b) 0.01 mg/mL to 0.6 mg/mL polysorbate 20 or polysorbate 80,
(c) 25 mM to 290 mM proline, and
(d) 10 mM to 50 mM acetate buffer, the pharmaceutical composition having a pH of 4.6 to 5.4.

In some embodiments, provided is the pharmaceutical composition according to any one of the above, which comprises the following components:
(a) 10 mg/mL to 80 mg/mL said anti-RANKL-NGF bispecific antibody,
(b) 0.01 mg/mL to 0.6 mg/mL polysorbate 80,
(c) 25 mM to 250 mM proline, and
(d) 10 mM to 50 mM acetate buffer, the pharmaceutical composition having a pH of 4.6 to 5.4.

In some embodiments, provided is the pharmaceutical composition according to any one of the above, which comprises the following components:
(a) 20 mg/mL to 80 mg/mL said anti-RANKL-NGF bispecific antibody,
(b) 0.01 mg/mL to 0.4 mg/mL polysorbate 80,
(c) 210 mM to 270 mM proline, and
(d) 10 mM to 30 mM acetic acid-sodium acetate buffer, the pharmaceutical composition having a pH of 4.6 to 5.4.

In some embodiments, provided is the pharmaceutical composition according to any one of the above, which comprises the following components:
(a) 63 mg/mL to 77 mg/mL said anti-RANKL-NGF bispecific antibody,
(b) 0.01 mg/mL to 0.2 mg/mL polysorbate 80,
(c) 210 mM to 270 mM proline, and
(d) 10 mM to 30 mM acetic acid-sodium acetate buffer, the pharmaceutical composition having a pH of 4.8 to 5.2.

In some embodiments, provided is the pharmaceutical composition according to any one of the above, which comprises the following components:
(a) 63 mg/mL to 77 mg/mL said anti-RANKL-NGF bispecific antibody,
(b) 0.01 mg/mL to 0.2 mg/mL polysorbate 80,
(c) 25 mM to 250 mM proline, and
(d) 10 mM to 20 mM acetic acid-sodium acetate buffer, the pharmaceutical composition having a pH of 4.8 to 5.2.

In some embodiments, provided is the pharmaceutical composition according to any one of the above, which comprises the following components:
(a) 63 mg/mL to 77 mg/mL said anti-RANKL-NGF bispecific antibody,
(b) 0.01 mg/mL to 0.2 mg/mL polysorbate 80,
(c) 240 mM proline, and
(d) 10 mM to 20 mM acetic acid-sodium acetate buffer; the pharmaceutical composition having a pH of 4.8 to 5.2.

In some embodiments, provided is the pharmaceutical composition according to any one of the above, which comprises the following components:
(a) 63 mg/mL to 77 mg/mL said anti-RANKL-NGF bispecific antibody,
(b) 0.05 mg/mL to 0.15 mg/mL polysorbate 80,
(c) 210 mM to 270 mM proline, and
(d) 16 mM to 24 mM acetic acid-sodium acetate buffer, the pharmaceutical composition having a pH of 4.8 to 5.2.

In some embodiments, provided is the pharmaceutical composition according to any one of the above, which comprises the following components:
(a) 63 mg/mL to 77 mg/mL said anti-RANKL-NGF bispecific antibody,
(b) 0.08 mg/mL to 0.12 mg/mL polysorbate 80,
(c) 210 mM to 270 mM proline, and
(d) 16 mM to 24 mM acetic acid-sodium acetate buffer, the pharmaceutical composition having a pH of 4.8 to 5.2.

In some embodiments, provided is the pharmaceutical composition according to any one of the above, which comprises the following components:
(a) about 70 mg/mL said anti-RANKL-NGF bispecific antibody,
(b) about 0.1 mg/mL polysorbate 80,
(c) about 240 mM proline, and
(d) about 20 mM acetic acid-sodium acetate buffer, the pharmaceutical composition having a pH of 4.8 to 5.2.

In some embodiments, provided is the pharmaceutical composition according to any one of the above, which comprises the following components:
(a) 70 mg/mL said anti-RANKL-NGF bispecific antibody,
(b) 0.1 mg/mL polysorbate 80,
(c) 240 mM proline, and
(d) 20 mM acetic acid-sodium acetate buffer, the pharmaceutical composition having a pH of 4.8 to 5.2.

In some embodiments, provided is the pharmaceutical composition described above, wherein the pharmaceutical composition is a liquid formulation. In some embodiments, the solvent of the liquid formulation is water.

The present disclosure further provides a lyophilized formulation, wherein the lyophilized formulation is capable of forming the pharmaceutical composition according to any one of the above after being reconstituted.

The present disclosure further provides a lyophilized formulation, which is a formulation in a lyophilized form of the pharmaceutical composition according to any one of the above.

The present disclosure further provides a method for preparing a lyophilized formulation, which comprises the step of lyophilizing the pharmaceutical composition according to any one of the above. In some embodiments, the lyophilization according to any one of the above comprises steps of pre-freezing, primary drying, and secondary drying in sequence.

The present disclosure further provides a lyophilized formulation, wherein the formulation is obtained by lyophilizing the pharmaceutical composition according to any one of the above.

The present disclosure further provides a reconstituted solution, wherein the reconstituted solution is obtained by reconstituting the lyophilized formulation according to any one of the above.

The present disclosure further provides a reconstituted solution, which is a formulation in a reconstituted form of the lyophilized formulation according to any one of the above. In some embodiments, provided is the reconstituted solution according to any one of the above, which has identical components and contents to those of the aforementioned pharmaceutical composition.

In some embodiments, provided is the reconstituted solution according to any one of the above, which comprises the following components:
(a) 1 mg/mL to 150 mg/mL said anti-RANKL-NGF bispecific antibody, (b) 0.01 mg/mL to 1.0 mg/mL surfactant, (c) 1 mM to 300 mM stabilizer, and (d) 5 mM to 100 mM buffer, the pharmaceutical composition having a pH of 4.2 to 7.0.

In some embodiments, provided is the reconstituted solution according to any one of the above, which comprises the following components:
(a) 1 mg/mL to 150 mg/mL said anti-RANKL-NGF bispecific antibody according to any one of the above, (b) 0.01 mg/mL to 1.0 mg/mL surfactant, (c) 1 mM to 300 mM proline, and (d) 5 mM to 100 mM buffer, the pharmaceutical composition having a pH of 4.2 to 7.0.

In some embodiments, provided is the reconstituted solution according to any one of the above, which comprises the following components:
(a) 10 mg/mL to 80 mg/mL said anti-RANKL-NGF bispecific antibody, (b) 0.01 mg/mL to 0.6 mg/mL polysorbate 20 or polysorbate 80, (c) 25 mM to 290 mM proline, and (d) 10 mM to 50 mM acetate buffer, the pharmaceutical composition having a pH of 4.6 to 5.4.

In some embodiments, provided is the reconstituted solution according to any one of the above, which comprises the following components:
(a) 10 mg/mL to 80 mg/mL said anti-RANKL-NGF bispecific antibody, (b) 0.01 mg/mL to 0.6 mg/mL polysorbate 80, (c) 25 mM to 250 mM proline, and (d) 10 mM to 50 mM acetate buffer, the pharmaceutical composition having a pH of 4.6 to 5.4.

In some embodiments, provided is the reconstituted solution according to any one of the above, which comprises the following components:
(a) 20 mg/mL to 80 mg/mL said anti-RANKL-NGF bispecific antibody, (b) 0.01 mg/mL to 0.4 mg/mL polysorbate 80, (c) 210 mM to 270 mM proline, and (d) 10 mM to 30 mM acetate buffer, the pharmaceutical composition having a pH of 4.6 to 5.4.

In some embodiments, provided is the reconstituted solution according to any one of the above, which comprises the following components:
(a) 63 mg/mL to 77 mg/mL said anti-RANKL-NGF bispecific antibody, (b) 0.01 mg/mL to 0.2 mg/mL polysorbate 80, (c) 210 mM to 270 mM proline, and (d) 10 mM to 30 mM acetic acid-sodium acetate buffer, the pharmaceutical composition having a pH of 4.8 to 5.2.

In some embodiments, provided is the reconstituted solution according to any one of the above, which comprises the following components:
(a) 63 mg/mL to 77 mg/mL said anti-RANKL-NGF bispecific antibody, (b) 0.01 mg/mL to 0.2 mg/mL polysorbate 80, (c) 25 mM to 250 mM proline, and (d) 10 mM to 20 mM of acetic acid-sodium acetate buffer, the pharmaceutical composition having a pH of 4.8 to 5.2.

In some embodiments, provided is the reconstituted solution according to any one of the above, which comprises the following components:
(a) 63 mg/mL to 77 mg/mL said anti-RANKL-NGF bispecific antibody, (b) 0.01 mg/mL to 0.2 mg/mL polysorbate 80, (c) 240 mM proline, and (d) 10 mM to 20 mM acetic acid-sodium acetate buffer, the pharmaceutical composition having a pH of 4.8 to 5.2.

In some embodiments, provided is the reconstituted solution according to any one of the above, which comprises the following components:
(a) 63 mg/mL to 77 mg/mL said anti-RANKL-NGF bispecific antibody, (b) 0.05 mg/mL to 0.15 mg/mL polysorbate 80, (c) 210 mM to 270 mM proline, and (d) 16 mM to 24 mM acetic acid-sodium acetate buffer, the pharmaceutical composition having a pH of 4.8 to 5.2.

In some embodiments, provided is the reconstituted solution according to any one of the above, which comprises the following components:
(a) 63 mg/mL to 77 mg/mL said anti-RANKL-NGF bispecific antibody, (b) 0.08 mg/mL to 0.12 mg/mL polysorbate 80, (c) 210 mM to 270 mM proline, and (d) 16 mM to 24 mM acetic acid-sodium acetate buffer, the pharmaceutical composition having a pH of 4.8 to 5.2.

In some embodiments, provided is the reconstituted solution according to any one of the above, which comprises the following components:
(a) about 70 mg/mL said anti-RANKL-NGF bispecific antibody, (b) about 0.1 mg/mL polysorbate 80, (c) about 240 mM proline, and (d) about 20 mM acetic acid-sodium acetate buffer, the pharmaceutical composition having a pH of 4.8 to 5.2.

In some embodiments, provided is the reconstituted solution according to any one of the above, which comprises the following components:
(a) 70 mg/mL said anti-RANKL-NGF bispecific antibody, (b) 0.1 mg/mL polysorbate 80, (c) 240 mM proline, and (d) 20 mM acetic acid-sodium acetate buffer, the pharmaceutical composition having a pH of 4.8 to 5.2.

In some embodiments, provided is the pharmaceutical composition or the reconstituted solution according to any one of the above, which is a formulation for subcutaneous, intravenous, intraperitoneal, or intramuscular injection. In some embodiments, provided is the pharmaceutical composition or the reconstituted solution according to any one of the above, which is a formulation for subcutaneous injection.

In some embodiments, provided is the pharmaceutical composition or the reconstituted solution according to any one of the above, which is suitable for subcutaneous, intravenous, intraperitoneal, or intramuscular injection; preferably, it is suitable for subcutaneous injection.

In some embodiments, provided is the pharmaceutical composition or the reconstituted solution or the lyophilized formulation according to any one of the above, which is used for preparing a medicament for subcutaneous, intravenous, intraperitoneal, or intramuscular injection; preferably, it is used for preparing a medicament for subcutaneous injection.

The present disclosure further provides a kit, comprising at least one container, wherein each container independently contains the pharmaceutical composition according to any one of the above, the lyophilized formulation according to any one of the above, or the reconstituted solution according to any one of the above.

In some embodiments, the present disclosure further provides a method for diagnosing, treating, and ameliorating a disorder in a subject, which comprises administering to the subject an effective amount of the pharmaceutical composition according to any one of the above, the lyophilized formulation according to any one of the above, the reconstituted solution according to any one of the above, or the kit according to any one of the above.

In some embodiments, the present disclosure further provides use of the pharmaceutical composition according to any one of the above, the lyophilized formulation according to any one of the above, the reconstituted solution according to any one of the above, or the kit according to any one of the above in the preparation of a medicament for treating or preventing a disease.

In some embodiments, the present disclosure further provides a method for treating or preventing a disease, which comprises administering to a subject a therapeutically effective amount of the pharmaceutical composition according to any one of the above, the lyophilized formulation according to any one of the above, the reconstituted solution according to any one of the above, or the kit according to any one of the above.

In some embodiments, the present disclosure further provides the pharmaceutical composition according to any one of the above, the lyophilized formulation according to any one of the above, the reconstituted solution according to any one of the above, or the kit according to any one of the above for use in the treatment or prevention of a disease.

In one aspect, the present disclosure further provides use of the pharmaceutical composition according to any one of the above, the lyophilized formulation according to any one of the above, the reconstituted solution according to any one of the above, or the kit according to any one of the above in the preparation of a medicament for preventing or treating a disease or disorder.

In some specific embodiments, the disease according to any one of the above is pain, joint stiffness, or bone loss.

In some embodiments, the pain is selected from the group consisting of osteoarticular pain, rheumatoid arthritis pain, gout, bone cancer pain, fracture pain, post-surgical pain, cancer pain, painful bladder syndrome, musculoskeletal pain, prostatitis (e.g., chronic prostatitis), pelvic pain (e.g., chronic pelvic pain), interstitial cystitis, lower back pain, dysmenorrhea, pain associated with bone disease, trigeminal neuralgia, post-herpetic neuralgia, herpes zoster infection, sciatica, migraine, diabetic neuropathy, and pain associated with peripheral nerves.

In some embodiments, the bone loss is associated with at least one condition selected from the group consisting of osteoporosis, Paget's disease, osteomyelitis, hypercalcemia, osteopenia, osteoporosis, osteonecrosis, bone injury, bone resorption, osteogenesis imperfecta, inflammation, autoimmune disease, enteritis, rheumatoid arthritis, systemic lupus erythematosus, Crohn's disease, periodontal bone resorption, osteolytic metastasis, and cancer.

In some embodiments, the cancer is selected from the group consisting of breast cancer, prostate cancer, thyroid cancer, renal cancer, lung cancer, esophageal cancer, rectal cancer, bladder cancer, cervical cancer, ovarian cancer, liver cancer, gastrointestinal cancer, melanoma, multiple myeloma, osteosarcoma, lymphoma, non-small cell lung cancer, bone tumor, and Hodgkin's disease.

In some embodiments, the aforementioned disease is an NGF- or RANKL-associated disease.

In some embodiments, the aforementioned disease is a disease that expresses NGF or RANKL.

In one aspect, the present disclosure provides a method for treating or preventing an NGF- or RANKL-associated disease, which comprises administering to a subject a prophylactically or therapeutically effective amount of the pharmaceutical composition according to any one of the above, the lyophilized formulation according to any one of the above, the reconstituted solution according to any one of the above, or the kit according to any one of the above.

In some embodiments, the NGF- or RANKL-associated disease is pain, joint stiffness, or bone loss.

In one aspect, the pharmaceutical composition according to any one of the above, the lyophilized formulation according to any one of the above, the reconstituted solution according to any one of the above, or the kit according to any one of the above of the present disclosure can be used as a medicament. In some embodiments, provided is a medicament for use in treating pain, joint stiffness, or bone loss. In some embodiments, provided is a medicament for use in treating osteoarticular pain, rheumatoid arthritis pain, gout, bone cancer pain, fracture pain, post-surgical pain, cancer pain, painful bladder syndrome, musculoskeletal pain, prostatitis (e.g., chronic prostatitis), pelvic pain (e.g., chronic pelvic pain), interstitial cystitis, lower back pain, dysmenorrhea, pain associated with bone disease, trigeminal neuralgia, post-herpetic neuralgia, herpes zoster infection, sciatica, migraine, diabetic neuropathy, and pain associated with peripheral nerves. In some embodiments, provided is a medicament for use in treating bone loss caused by osteoporosis, Paget's disease, osteomyelitis, hypercalcemia, osteopenia, osteoporosis, osteonecrosis, bone injury, bone resorption, osteogenesis imperfecta, inflammation, autoimmune disease, enteritis, rheumatoid arthritis, systemic lupus erythematosus, Crohn's disease, periodontal bone resorption, osteolytic metastasis, and cancer.

In one aspect, the present disclosure provides use of the pharmaceutical composition according to any one of the above, the lyophilized formulation according to any one of the above, the reconstituted solution according to any one of the above, or the kit according to any one of the above in the preparation of a medicament for preventing or treating an NGF- or RANKL-associated disease. In some embodiments, the NGF- or RANKL-associated disease is pain, joint stiffness, or bone loss.

In one aspect, the pharmaceutical composition according to any one of the above, the lyophilized formulation according to any one of the above, the reconstituted solution according to any one of the above, or the kit according to any one of the above provided by the present disclosure can be used as a medicament for preventing or treating an NGF- or RANKL-associated disease. In some embodiments, the NGF- or RANKL-associated disease is pain, joint stiffness, or bone loss.

### BRIEF DESCRIPTION OF THE DRAWINGS

FIG. 1: a diagram of the DVD-IgG structure.

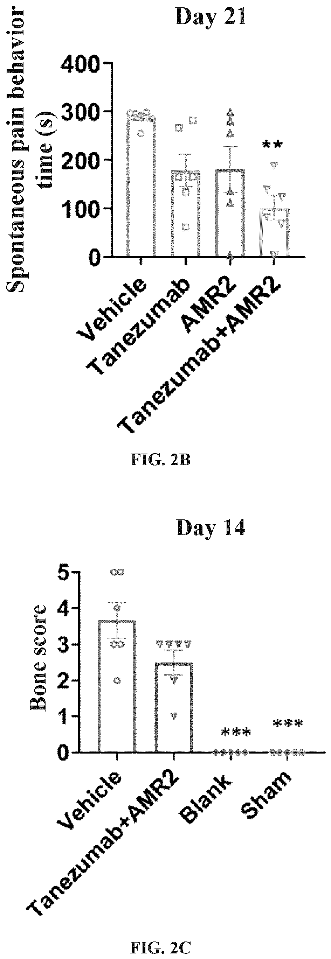

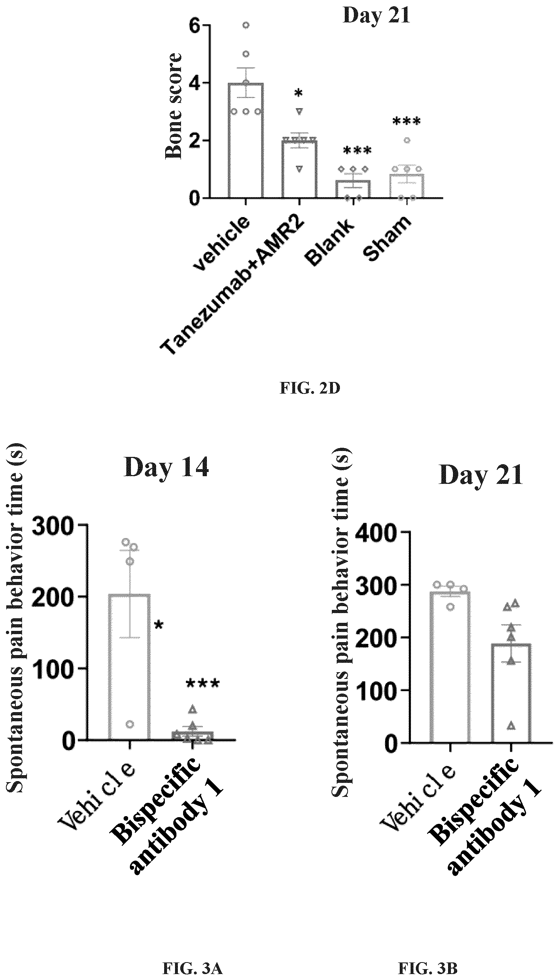

FIG. 2A to FIG. 2D: FIG. 2A shows statistical results of pain behaviors in mice on day 14 of combination therapy; FIG. 2B shows statistical results of pain behaviors in mice on day 21 of combination therapy; FIG. 2C shows bone injury scores of mice on day 14 of combination therapy; FIG. 2D shows bone injury scores of mice on day 21 of combination therapy; wherein vs vehicle **** represents vs vehicle, P < 0.0001; *** represents vs vehicle, P < 0.001; ** represents vs vehicle, P < 0.01; * represents vs vehicle, P < 0.05. Blank; Sham; vehicle.
FIG. 3A to FIG. 3B: FIG. 3A shows statistical results of pain behaviors in mice on day 14 of using bispecific antibody 1; FIG. 3B shows statistical results of pain behaviors in mice on day 21 of using bispecific antibody 1; wherein vs vehicle **** represents vs vehicle, P < 0.0001; *** represents vs vehicle, P < 0.001; ** represents vs vehicle, P < 0.01; * represents vs vehicle, P < 0.05. Vehicle.

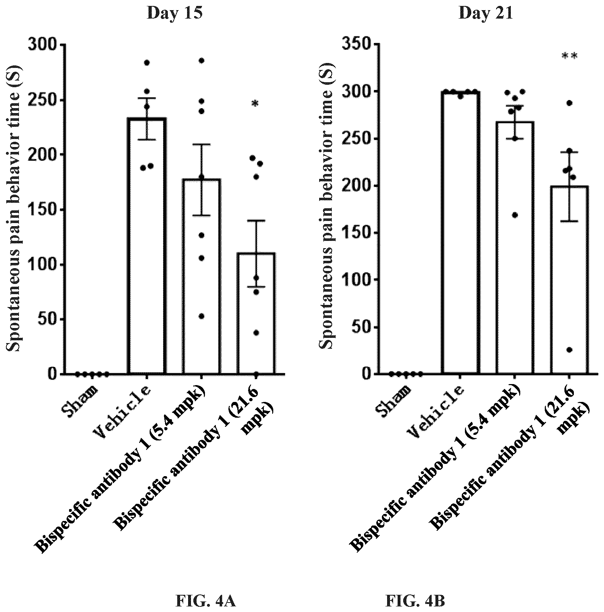

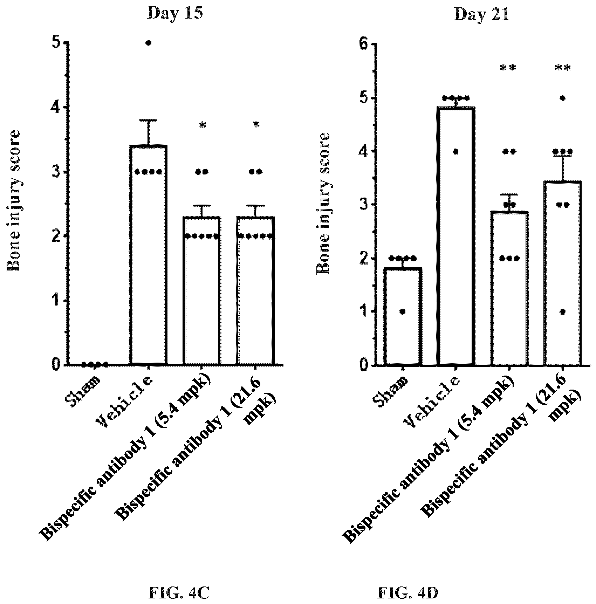

FIG. 4A to FIG. 4D: FIG. 4A shows statistical results of pain behaviors in mice on day 15 of using bispecific antibody 1; FIG. 4B shows statistical results of pain behaviors in mice on day 21 of using bispecific antibody 1; FIG. 4C shows bone injury scores of mice on day 15 of using bispecific antibody 1; FIG. 4D shows bone injury scores of mice on day 21 of using bispecific antibody 1; wherein vs vehicle **** represents vs vehicle, P < 0.0001; *** represents vs vehicle, P < 0.001; ** represents vs vehicle, P < 0.01; * represents vs vehicle, P < 0.05. Sham; vehicle.

### DETAILED DESCRIPTION

### Terminology

In order to facilitate the understanding of the present disclosure, certain technical and scientific terms are specifically defined below. Unless otherwise explicitly defined herein, all other technical and scientific terms used herein have the meanings generally understood by those of ordinary skill in the art to which the present disclosure belongs. As used in the present disclosure, the singular forms "a", "an", and "the" include plural referents unless the context clearly dictates otherwise.

Unless otherwise clearly stated in the context, throughout the description and the claims, the words "comprise", "have", "include", and the like, should be construed in an inclusive sense as opposed to an exclusive or exhaustive sense.

"Optional" or "optionally" means that the event or circumstance subsequently described may, but does not necessarily, occur, and this description includes instances where the event or circumstance occurs or does not occur.

It should be understood by those skilled in the art that when used with reference to numerical ranges, cutoff values, or specific values, "about" may mean within 1 or more than 1 standard deviation. Alternatively, "about" may mean a range up to 20% apart (i.e., ±20%). Since many of the numerical values used herein are determined experimentally, those skilled in the art will appreciate that such determinations can and typically do vary from experiment to experiment. Because of this inherent difference, it is believed that the values used herein should not be unduly limited. Thus, the term "about" is used to encompass variations of ±20% or less, variations of ±10% or less, variations of ±5% or less, variations of ±1% or less, variations of ±0.5% or less, or variations of ±0.1% or less from a specified value.

While the present disclosure provides content ranges or values, those of ordinary skill in the art will understand that the content ranges or values encompass acceptable margins of error for the specific value determined.

The three-letter and single-letter codes for amino acids used herein are as described in J. biol. chem. 243, p3558 (1968).

"Anti-RANKL antibody" refers to an antibody that is capable of binding to RANKL or an epitope thereof and inhibiting the biological activity of RANKL and/or inhibiting downstream pathways of RANKL.

The term "NGF" or nerve growth factor refers to a nerve growth factor and variants thereof that retain at least part of the biological activity of NGF. As used herein, NGF includes the wild-type sequence NGF or naturally occurring variants thereof of all mammalian species, including human, murine, monkey, canine, feline, equine, or bovine.

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a structure different from the general chemical structure of amino acids, but function in a manner similar to naturally occurring amino acids.

"Antibody" is used in the broadest sense and encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. "Natural antibody" refers to a naturally occurring immunoglobulin molecule. For example, a native IgG antibody is a heterotetrameric glycoprotein of about 150,000 Daltons composed of two identical light chains and two identical heavy chains linked by a disulfide bond. From the N-terminus to the C-terminus, each heavy chain has one variable region (VH), also known as variable heavy domain or heavy chain variable region, followed by three constant domains (CH1, CH2, and CH3). Similarly, from the N-terminus to the C-terminus, each light chain has one variable region (VL), also known as variable light domain or light chain variable domain, followed by one constant light domain (light chain constant region, CL). The term "bispecific antibody" refers to an antibody (including an antibody or an antigen-binding fragment thereof, such as a single-chain antibody) capable of binding specifically to two different antigens or at least two different epitopes of the same antigen.

The term "variable region" or "variable domain" refers to a domain in an antibody heavy or light chain that is involved in the binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable region that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Each VH and VL is composed of three CDRs and four FRs arranged from the amino terminus to the carboxyl terminus in the following order: FR1, CDR1, FR2, CDR2, FR3, CDR3, and FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol., Oct. 16, 2018; 9: 2278), and the like. The corresponding relationships between the various numbering schemes are well known to those skilled in the art. The numbering schemes of the present disclosure are shown in Table 1 below.

**Table 1. Relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable region and CDR sequences in examples of the present disclosure. Although the Kabat numbering scheme is employed to define amino acid residues in specific embodiments, corresponding technical solutions for other numbering schemes are to be considered as equivalent technical solutions.

"Pharmaceutical composition" means that it comprises one or more bispecific antibodies as described herein, as well as other components such as physiologically/pharmaceutically acceptable carriers and excipients. The pharmaceutical composition is intended to promote administration to an organism and facilitate the absorption of the active ingredient so that it can exert its biological activity. As used herein, "pharmaceutical composition" and "formulation" are not mutually exclusive.

"Effective amount" comprises an amount sufficient to ameliorate or prevent a symptom or sign of a medical disorder. An effective amount also means an amount sufficient to allow or facilitate diagnosis. The effective amount for a particular subject or veterinary subject may vary depending on factors such as the disorder to be treated, the general health of the subject, the method and route and dosage of administration, and the severity of side effects. An effective amount may be the maximum dose or administration regimen to avoid significant side effects or toxic effects.

"Pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as phosphate-buffered saline solutions, water, emulsions such as oil/water emulsions, and various types of wetting agents. In some examples, the diluent for aerosol or parenteral administration is phosphate-buffered saline (PBS) or normal (0.9%) saline. Compositions comprising such carriers are formulated by well-known conventional methods.

"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine. Examples of histidine buffers include histidine-histidine hydrochloride, histidine-histidine acetate, histidine-histidine phosphate, histidine-histidine sulfate, and the like. The histidine-histidine hydrochloride buffer is preferred. The histidine-histidine hydrochloride buffer may be prepared from histidine and hydrochloric acid, or from histidine and histidine hydrochloride.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer that comprises succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Phosphate buffer" is a buffer comprising phosphate ions. Examples of phosphate buffers include citric acid-disodium hydrogen phosphate, disodium hydrogen phosphate-sodium dihydrogen phosphate, disodium hydrogen phosphate-potassium dihydrogen phosphate, disodium hydrogen phosphate-citric acid, and the like. The preferred phosphate buffer is citric acid-disodium hydrogen phosphate.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

Poloxamers are block copolymers of ethylene oxide and propylene oxide, are water-soluble, and are used as surfactants in pharmaceutical formulations. Examples of poloxamers include poloxamer 188.

"Lyophilized formulation" refers to a formulation or a pharmaceutical composition obtained by lyophilizing a pharmaceutical composition or a formulation in liquid or solution form in vacuum.

The pharmaceutical composition described herein can achieve a stable effect, that is, the antibody in the pharmaceutical composition substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable formulation is one in which no significant change is observed under the following conditions: stored at refrigeration temperature (2-8 °C) for at least 3 months, preferably 6 months, more preferably 1 year, and even more preferably up to 2 years. **In** addition, a stable liquid formulation includes a liquid formulation that exhibits desirable features after storage at temperatures including 25 °C for periods including 2 weeks, 4 weeks, 1 month, 3 months, or 6 months. In addition, a stable liquid formulation further includes a liquid formulation that exhibits desirable features after storage at a temperature of 40 °C for periods including 2 weeks, 4 weeks, 1 month, 3 months, or 6 months. Typical examples for stability are as follows: generally, no more than about 10%, preferably no more than about 5%, of antibodies aggregate or are degraded as measured by SEC-HPLC. The formulation is a pale yellow, nearly colorless and clear liquid, or a colorless and clear liquid, or is clear to slightly opalescent, by visual analysis. The concentration, pH, and osmolality of the formulation have changes of no more than ±10%, preferably changes of no more than ±5%. Generally, aggregation of no more than about 10%, preferably no more than about 5% is formed in the formulation.

An antibody "retains its physical stability" in a pharmaceutical formulation if it shows no significant increase in aggregation, precipitation, and/or denaturation upon visual inspection of color and/or clarity, or as determined by UV light scattering, size exclusion chromatography (SEC), and dynamic light scattering (DLS). Changes in protein conformation can be evaluated by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be evaluated by detecting and quantifying chemically changed proteins. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

An antibody "retains its biological activity" in a pharmaceutical formulation if the biological activity of the antibody at a given time is within a predetermined range of the biological activity exhibited during the preparation of the pharmaceutical formulation.

"Administrating", "giving", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administering", "giving", and "treating" can refer to, for example, therapeutic, pharmacokinetic, diagnostic, research, and experimental methods. The treatment of cells comprises contacting a reagent with cells and contacting the reagent with a fluid, where the fluid is in contact with the cells. "Administering", "giving", and "treating" also refer to treating, e.g., cells by reagents, diagnosis, binding compositions or by another cell *in vitro* and *ex vivo.* "Treating", when applied to humans, veterinary, or research subjects, refers to therapeutic treatment, preventive or prophylactic measures, and research and diagnostic applications.

"Treatment" refers to administering, either internally or externally, a therapeutic agent, for example, comprising any one of the pharmaceutical compositions of the present disclosure, to a patient with one or more symptoms of a disease on which the therapeutic agent is known to have a therapeutic effect. Generally, the therapeutic agent is administered in an amount effective to alleviate one or more disease symptoms in the patient or population being treated to induce regression of such symptoms or to inhibit the development of such symptoms to any clinically measurable degree. The amount of the therapeutic agent effective to alleviate any specific disease symptom (also known as a "therapeutically effective amount") may vary depending on a variety of factors, such as the disease state, age and body weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although an embodiment of the present disclosure (for example, a treatment method or a product) may not be effective in alleviating every target disease symptom, it should alleviate the target disease symptom in a statistically significant number of patients as determined by any statistical testing methods known in the art, such as Student t-test, chi-square test, Mann and Whitney's U test, Kruskal-Wallis test (H test), Jonckheere-Terpstra test, and Wilcoxon test.

The pharmaceutical composition of the present disclosure may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection. Various administration schedules are contemplated herein, including but not limited to, single administration or multiple administrations at multiple time points, bolus administration, and pulse infusion. In some embodiments, the pharmaceutical composition of the present disclosure is administered by subcutaneous injection.

The pharmaceutical composition of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the method of administration, the timing of administration, and other factors known to medical practitioners. Optionally, the pharmaceutical composition may also be formulated with one or more additional agents used for preventing or treating the disorder. The effective amount of such additional agents depends on the amount of the antigen-binding molecule present in the pharmaceutical composition, the type of the disorder or treatment, and other factors. Such additional agents may be used in the same doses and with the same routes of administration as described herein, or in about 1% to 99% of the doses described herein, or in any dose and by any route that is empirically/clinically determined to be appropriate.

There is no limitation for an NGF- or RANKL-associated disease in the present disclosure, as long as it is a disease associated with NGF or RANKL. For example, a therapeutic response induced by the antibody of the present disclosure can be achieved by binding to human NGF or RANKL, then blocking the binding of NGF or RANKL to its receptor, or killing cells overexpressing NGF or RANKL; or inhibiting the growth of cells overexpressing NGF or RANKL.

The details of one or more embodiments of the present disclosure are set forth in the specification above. Although any methods and materials similar or identical to those described herein can be used in the practice or testing of the present disclosure, the preferred methods and materials are described below. Other features, objects, and advantages of the present disclosure will be apparent from the specification and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure, which is defined by the claims.

### Examples-Preparation and Detection of Anti-RANKL-NGF Bispecific Antibody

PCT/CN2022/092333 (filing date: May 12, 2022; priority Patent Application No.: CN202110515444.9) is incorporated herein by reference in its entirety.

### Example 1: Preparation of Anti-RANKL Antibodies

Primers were designed and yeast libraries were constructed. The libraries were enriched and screened for candidate clones using the RANKL protein (Sino biological, 11682-HNCH), and the amino acid sequences of the light and heavy chain variable regions of antibodies in the clones were determined by sequencing. The sequences of the obtained exemplary anti-RANKL antibody are as follows:

**Table 2. CDR region sequences of anti-RANKL antibody**

| | | | | |
|---|---|---|---|---|
| D75H | HCDR1 | SYAMS SEQ ID NO: 1 | LCDR1 | RASQSVRGRYLA SEQ ID NO: 4 |
| | HCDR2 | GITGSGGSTYYADSVKG SEQ ID NO: 2 | LCDR2 | GASSRAT SEQ ID NO: 5 |
| | HCDR3 | DPGTTSIMSWFDP SEQ ID NO: 3 | LCDR3 | QQYGSSPRT SEQ ID NO: 6 |

**Table 3. Variable region sequences of RANKL antibody**

| Antibody name | Heavy chain variable region sequence | Light chain variable region sequence |
|---|---|---|
| D75H | EVQLLESGGGLVQPGGSLRLSC AASGFTFSSYAMSWVRQAPGK GLEWVSGITGSGGSTYYADSVK GRFTISRDNSKNTLYLQMNSLR AEDTAVYYCVKDPGTTSIMSWF DPWGQGTLVTVSS SEQ ID NO: 7 | EIVLTQSPGTLSLSPGERATLSCR ASQSVRGRYLAWYQQKPGQAP RLLIYGASSRATGIPDRFSGSGSG TDFTLTISRLEPEDFAVFYCQQY GSSPRTFGQGTKVEIK SEQ ID NO: 8 |

The above variable regions were fused to a human light chain constant region and a human heavy chain constant region to form complete antibody light and heavy chains. The constant region sequences of an exemplary antibody are as follows:
Human IgG4 heavy chain constant region: 
ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K
SEQ ID NO: 9;
Human κ light chain constant region:



SEQ ID NO: 10.

The sequences of the exemplary anti-RANKL antibody are as follows:
D75H heavy chain: 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCVKDPGTTSIMS
WFDPWGQGTLVTVSSASTKGPSVFPLAPCSRSTSESTAALGCLVKDYFPEPVTVSWN
 
SGALTSGVHTFPAVLQSSGLYSLSSVVTVPSSSLGTKTYTCNVDHKPSNTKVDKRVESK
YGPPCPPCPAPEFLGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKT
ISKAKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLGK
SEQ ID NO: 11;
D75H light chain:

EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID NO: 12.

The sequences of the control anti-RANKL antibody are as follows:
Denosumab heavy chain sequence: 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCAKDPGTTVIMS
WFDPWGQGTLVTVSSASTKGPSVFPLAPCSRSTSESTAALGCLVKDYFPEPVTVS
WNSGALTSGVHTFPAVLQSSGLYSLSSVVTVPSSNFGTQTYTCNVDHKPSNTKV
DKTVERKCCVECPPCPAPPVAGPSVFLFPPKPKDTLMISRTPEVTCVVVDVSHED
PEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIA
VEWESNGQPENNYKTTPPMLDSDGSFFLYSKLTVDKSRWQQGNVFSCSVMHEA
LHNHYTQKSLSLSPGK
SEQ ID NO: 13;
Denosumab light chain sequence:

EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 14.

### Example 2: Preparation of Anti-RANKL/NGF Bispecific Antibody

The second antigen-binding domain of the bispecific antibody of the present disclosure that binds to NGF can be derived from the antigen-binding moiety of any suitable antibody. Particularly suitable antibodies are described, for example, in the international application WO2004058184A2 (incorporated herein by reference in its entirety).

The sequences of the CDRs and variable regions of the NGF binding domain of the exemplary bispecific antibody are shown below:

**Table 4. CDRs of NGF binding domain**

| | | | |
|---|---|---|---|
| HCDR1 | GYDLN (SEQ ID NO: 15) | LCDR1 | RASQSISNNLN (SEQ ID NO: 18) |
| HCDR2 | IIWGDGTTDYNSAVKS (SEQ ID NO: 16) | LCDR2 | YTSRFHS (SEQ ID NO: 19) |
| HCDR3 | GGYWYATSYYFDY (SEQ ID NO: 17) | LCDR3 | QQEHTLPYT (SEQ ID NO: 20) |

NGF binding domain heavy chain variable region: 



SEQ ID NO: 21;
NGF binding domain light chain variable region:



SEQ ID NO: 22.

The variable regions of the NGF binding domain were fused to a heavy chain constant region of SEQ ID NO: 9 or SEQ ID NO: 23 and a light chain constant region of SEQ ID NO: 10, respectively, to obtain an anti-NGF antibody. The sequences are as follows: 
ASTKGPSVFPLAPCSRSTSESTAALGCLVKDYFPEPVTVSWNSGALTSGVHTFPA
VLQSSGLYSLSSVVTVPSSNFGTQTYTCNVDHKPSNTKVDKTVERKCCVECPPC
PAPPVAGPSVFLFPPKPKDTLMISRTPEVTCVVVDVSHEDPEVQFNWYVDGVEV
HNAKTKPREEQFNSTFRVVSVLTVVHQDWLNGKEYKCKVSNKGLPSSIEKTISK
TKGQPREPQVYTLPPSREEMTKNQVSLTCLVKGFYPSDIAVEWESNGQPENNYK
TTPPMLDSDGSFFLYSKLTVDKSRWQQGNVFSCSVMHEALHNHYTQKSLSLSP
GK
SEQ ID NO: 23;
anti-NGF antibody (N-mAb) heavy chain: 
QVQLQESGPGLVKPSETLSLTCTVSGFSLIGYDLNWIRQPPGKGLEWIGIIWGDG
TTDYNSAVKSRVTISKDTSKNQFSLKLSSVTAADTAVYYCARGGYWYATSYYFD
YWGQGTLVTVSSASTKGPSVFPLAPCSRSTSESTAALGCLVKDYFPEPVTVSWNSGA
LTSGVHTFPAVLQSSGLYSLSSVVTVPSSSLGTKTYTCNVDHKPSNTKVDKRVESKYGP
PCPPCPAPEFLGGPSVFLFPPKPKDTLMISRTPEVTCVVVDVSQEDPEVQFNWYVD
GVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSLSLGK
SEQ ID NO: 24;
anti-NGF antibody (N-mAb) light chain: DIQMTQSPSSLSASVGDRVTITCRASQSISNNLNWYQQKPGKAPKLLIYYTSRFH 
SGVPSRFSGSGSGTDFTFTISSLQPEDIATYYCQQEHTLPYTFGQGTKLEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC
SEQ ID NO: 25;
the sequences of the control anti-NGF antibody:
   Tanezumab heavy chain: 
QVQLQESGPGLVKPSETLSLTCTVSGFSLIGYDLNWIRQPPGKGLEWIGIIWGDG
TTDYNSAVKSRVTISKDTSKNQFSLKLSSVTAADTAVYYCARGGYWYATSYYFD
YWGQGTLVTVSSASTKGPSVFPLAPCSRSTSESTAALGCLVKDYFPEPVTVSWNSGA
LTSGVHTFPAVLQSSGLYSLSSVVTVPSSNFGTQTYTCNVDHKPSNTKVDKTVERKCC
VECPPCPAPPVAGPSVFLFPPKPKDTLMISRTPEVTCVVVDVSHEDPEVQFNWYVDG
VEVHNAKTKPREEQFNSTFRVVSVLTVVHQDWLNGKEYKCKVSNKGLPSSIEKTISKT
KGQPREPQVYTLPPSREEMTKNQVSLTCLVKGFYPSDIAVEWESNGQPENNYKTTPP
MLDSDGSFFLYSKLTVDKSRWQQGNVFSCSVMHEALHNHYTQKSLSLSPGK
SEQ ID NO: 26;
   Tanezumab light chain:

DIQMTQSPSSLSASVGDRVTITCRASQSISNNLNWYQQKPGKAPKLLIYYTSRFH
SGVPSRFSGSGSGTDFTFTISSLQPEDIATYYCQQEHTLPYTFGQGTKLEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC
SEQ ID NO: 27.

For the bone metastasis indication, monoclonal antibodies targeting NGF differ considerably from those targeting RANKL in clinical dosage at present. Therefore, in preparing a bispecific antibody against NGF and RANKL, it is necessary to balance the activities of the NGF arm and RANKL arm of the bispecific antibody so as to determine an appropriate dosage of the bispecific antibody, which will not cause the side effects arising from excessively high dosages or fail to fulfill the function of the bispecific antibody as excessively low dosages.

The bispecific antibody prepared by the present disclosure has a DVD-IgG structure (as shown in FIG. 1). The light chain variable domains (VLs) from the anti-RANKL antibody and the anti-NGF antibody were linked in tandem, directly or via a short linker by recombinant DNA techniques, followed by the light chain constant domain. Similarly, the heavy chain comprises 2 heavy chain variable domains (VHs) linked in tandem, followed by the constant domain CH1 and Fc region. The linker includes, but is not limited to, the following peptide linkers: ASTKGP (SEQ ID NO: 28) and TVAAP (SEQ ID NO: 29).

The sequences of the exemplary bispecific antibody are as follows:
First chain of bispecific antibody 1 
EVQLLESGGGLVQPGGSLRLSCAASGFTFSSYAMSWVRQAPGKGLEWVSGITG
SGGSTYYADSVKGRFTISRDNSKNTLYLQMNSLRAEDTAVYYCVKDPGTTSIMS
 
WFDPWGQGTLVTVSSASTKGPQVQLQESGPGLVKPSETLSLTCTVSGFSLIGYD
LNWIRQPPGKGLEWIGIIWGDGTTDYNSAVKSRVTISKDTSKNQFSLKLSSVTAA
DTAVYYCARGGYWYATSYYFDYWGQGTLVTVSSASTKGPSVFPLAPCSRSTSES
TAALGCLVKDYFPEPVTVSWNSGALTSGVHTFPAVLQSSGLYSLSSVVTVPSSSL
GTKTYTCNVDHKPSNTKVDKRVESKYGPPCPPCPAPEFLGGPSVFLFPPKPKDTL
MISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLYSRLTVD
KSRWQEGNVFSCSVMHEALHNHYTQKSLSLSLGK
SEQ ID NO: 30;
second chain of bispecific antibody 1

EIVLTQSPGTLSLSPGERATLSCRASQSVRGRYLAWYQQKPGQAPRLLIYGASSR
ATGIPDRFSGSGSGTDFTLTISRLEPEDFAVFYCQQYGSSPRTFGQGTKVEIKTVA
APDIQMTQSPSSLSASVGDRVTITCRASQSISNNLNWYQQKPGKAPKLLIYYTSR
FHSGVPSRFSGSGSGTDFTFTISSLQPEDIATYYCQQEHTLPYTFGQGTKLEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 31.

Note: The sequences single-underlined are RANKL antibody variable regions, the sequences double-underlined are NGF antibody variable regions, the sequences in bold type are linkers, and the others are constant regions.

### Test Examples

### Test Example 1: Tests of Anti-RANKL Antibodies for Affinity

A biosensor chip Protein A (GE, 29127556) was used to affinity-capture a certain amount of test antibody, and then antigens, the human RANKL protein (Sino Biological, 11682-HNCH) and the human NGF protein (Sino biological, 11050-HNAC), at a series of concentrations were allowed to flow over the surface of the chip. Reaction signals were detected in real time by using Biacore, and binding and dissociation curves were obtained. After dissociation was complete in each cycle, the biochip was washed and regenerated with a glycine-hydrochloric acid regeneration solution (GE, BR-1003-54) having a pH of 1.5. Fitting was performed on the experimental data using BIAevaluation version 4.1 software with a 1:1 model, and affinity values were obtained. The results are shown in Table 5 below.

**Table 5. Affinity KD values of anti-RANKL antibodies**

| Antibody | Affinity for human RANKL | | | |
|---|---|---|---|---|
| | ka (1/Ms) | kd (1/s) | KD (M) | Ratio of affinities (fold increase in affinity relative to denosumab) |
| D75H | 5.24E+04 | 7.73E-05 | 1.48E-09 | 6.24 |
| Denosumab | 1.90E+04 | 1.75E-04 | 9.24E-09 | 1.00 |

The results show that the affinity of the engineered antibody D75H is more than 6 times higher than that of denosumab.

### Test Example 2: Tests of Anti-RANKL Antibodies for Abilities to Block Binding of RANKL to RANK

The activity of antibodies blocking the binding of the ligand to the receptor was determined by ELISA. The human RANK protein was diluted to 2 µg/mL with a PBS buffer (BasalMedia, B320) at pH 7.4 and added to a 96-well microplate (Corning, 3590) at 100 µL/well, and the plate was incubated overnight at 4 °C. After the liquid was discarded, 1% Casein blocking solution (Thermo, 37528) was added at 200 µL/well for blocking, and the plate was incubated at 37 °C for 2 h. After the blocking was complete, the blocking solution was discarded, and the plate was washed 3 times with a PBST buffer (pH 7.4 PBS containing 0.1% tween-20) for later use. After a fixed concentration of biotin-labeled human RANKL protein (Sino Biological, 11682-HNCH) was mixed with an antibody serially diluted, the mixtures were pre-incubated at 37 °C for 30 min and then added to the blocked microplate. The plate was incubated at 37 °C for 1.5 h. After the incubation was complete, the plate was washed 3 times with PBST. Streptavidin-HRP (Invitrogen, 434323, diluted in a 1:4000 ratio) was added at 100 µL/well, and the plate was incubated at 37 °C for 1 h. The supernatant was removed. After the plate was washed 3 times with PBST, TMB chromogenic substrate (KPL, 5120-0077) was added at 100 µL/well, and the plate was incubated at room temperature for 10 min to 15 min. 1 M H₂SO₄ was added at 50 µL/well to stop the reactions. The absorbance values at 450 nm were measured using a microplate reader. A curve for the inhibition of the binding of the ligand to the receptor was fitted using software, and IC₅₀ values were calculated. The results are shown in Table 6 below.

**Table 6. Abilities of anti-RANKL antibodies to block binding of RANKL to RANK**

| Antibody name | Blocking binding of RANKL to RANK IC₅₀ (nM) |
|---|---|
| D75H | 0.2964 |
| Denosumab | 0.5324 |

The results show that the anti-RANKL antibody D75H can block the binding of RANKL to RANK, and its blocking activity is better than that of denosumab.

### Test Example 3: Determination of Binding Activity of Bispecific Antibody for Antigens of Different Species by ELISA

The binding activity of the bispecific antibody of the present disclosure for human RANKL (Sino biological, 11682-HNCH), monkey RANKL (Sino Biological, 90301-C01H), murine RANKL (R&D Systems, 462-TR/CF) and human NGF (the monkey NGF and the human NGF are identical in sequence) (Sino biological, 11050-HNAC), and murine NGF (Sino Biological, 50385-MNAC) was determined by ELISA as follows:
The antigen was diluted to 1 µg/mL with a PBS buffer (BasalMedia, B320) at pH 7.4 and added to a 96-well microplate (Corning, 9018) at 100 µL/well, and the plate was incubated overnight at 4 °C. After the liquid was discarded, a 5% skim milk (BD, 232100) blocking solution diluted with PBS was added at 300 µL/well, and the plate was incubated at 37 °C for 2 h for blocking. After the blocking was complete, the blocking solution was discarded, and the plate was washed 3 times with a PBST buffer (pH 7.4 PBS containing 0.1% tween-20). Then a bispecific antibody diluted with a sample diluent (pH 7.4 PBS containing 1% BSA) to different concentrations was added at 100 µL/well, and the plate was incubated in a constant-temperature incubator at 37 °C for 1 h. After the incubation was complete, the plate was washed 3 times with PBST. An HRP-labeled anti-human Fc secondary antibody (Abcam, ab97225) diluted with the sample diluent was added at 100 µL/well, and the plate was incubated at 37 °C for 1 h. After the plate was washed 3 times with PBST, TMB chromogenic substrate (KPL, 5120-0077) was added at 100 µL/well, and the plate was incubated at room temperature for 10 min to 15 min. 1 M H₂SO₄ was added at 50 µL/well to stop the reactions. The absorbance values at 450 nm were measured using a microplate reader, and the EC₅₀ values for binding of the bispecific antibody to the antigens were calculated. The results are shown in Table 7 below.

**Table 7. EC₅₀ for binding of bispecific antibody to antigens of different species**

| Antibody | ELISA EC₅₀ (nM) | | | | |
|---|---|---|---|---|---|
| | Human RANKL | Monkey RANKL | Murine RANKL | Human (monkey) NGF | Murine NGF |
| Bispecific antibody 1 | 0.06245 | 0.4666 | No binding | 12.27 | 4.344 |
| Denosumab | 0.1277 | 2.129 | No binding | No binding | No binding |
| Tanezumab | No binding | No binding | No binding | 2.270 | 1.151 |

The results show that the binding activity of bispecific antibody 1 for both human RANKL and monkey RANKL is better than that of the control antibody denosumab. However, neither bispecific antibody 1 nor denosumab cross-bound to murine RANKL. The binding activity of bispecific antibody 1 for both human NGF and murine NGF is significantly weaker than that of tanezumab, which indicates that bispecific antibody 1 of the present disclosure had successfully reduced the binding activity of the tanezumab end (the second antigen-binding domain that specifically binds to NGF).

### Test Example 4: Test of Bispecific Antibody for Affinity

A biosensor chip Protein A (GE, 29127556) was used to affinity-capture a certain amount of test antibody, and then antigens, the human RANKL protein (Sino Biological, 11682-HNCH) and the human NGF protein (Sino biological, 11050-HNAC), at a series of concentrations were allowed to flow over the surface of the chip. Reaction signals were detected in real time by using Biacore, and binding and dissociation curves were obtained. After dissociation was complete in each cycle, the biochip was washed and regenerated with a glycine-hydrochloric acid regeneration solution (GE, BR-1003-54) having a pH of 1.5. Fitting was performed on the experimental data using BIAevaluation version 4.1 software with a 1:1 model, and affinity values were obtained. The results are shown in Table 8 below.

**Table 8. Affinities of bispecific antibody for different antigens**

| Antibody | Affinity for human RANKL | | | Affinity for human NGF | | |
|---|---|---|---|---|---|---|
| | ka (1/Ms) | kd (1/s) | KD (M) | ka (1/Ms) | kd (1/s) | KD (M) |
| Bispecific antibody 1 | 2.25E+04 | 8.77E-05 | 3.89E-09 | 1.13E+03 | 6.33E-05 | 5.61E-08 |
| Denosumab | 1.04E+04 | 1.53E-04 | 1.47E-08 | / | / | / |
| Tanezumab | / | / | / | 1.29E+06 | 1.84E-04 | 1.42E-10 |

The results show that the affinity of bispecific antibody 1 for human RANKL is 3.8 times that of denosumab, and the affinity of bispecific antibody 1 for human NGF is significantly lower than that of tanezumab.

### Test Example 5: Blocking Experiments of Ligands and Receptors

The blocking activity of antibodies for ligands and receptors was determined by ELISA. A receptor protein (RANK or TrkA) was diluted to 2 µg/mL with a PBS buffer (BasalMedia, B320) at pH 7.4 and added to a 96-well microplate (Corning, 3590) at 100 µL/well, and the plate was incubated overnight at 4 °C. After the liquid was discarded, 1% Casein blocking solution (Thermo, 37528) was added at 200 µL/well for blocking, and the plate was incubated at 37 °C for 2 h. After the blocking was complete, the blocking solution was discarded, and the plate was washed 3 times with a PBST buffer (pH 7.4 PBS containing 0.1% tween-20) for later use. After a fixed concentration of biotin-labeled ligand protein (RANKL or NGF) was mixed with an antibody or fusion protein serially diluted, the mixtures were pre-incubated at 37 °C for 30 min and then added to the blocked microplate. The plate was incubated at 37 °C for 1.5 h. After the incubation was complete, the plate was washed 3 times with PBST. Streptavidin-HRP (Invitrogen, 434323, diluted in a 1:4000 ratio) was added at 100 µL/well, and the plate was incubated at 37 °C for 1 h. The supernatant was removed. After the plate was washed 3 times with PBST, TMB chromogenic substrate (KPL, 5120-0077) was added at 100 µL/well, and the plate was incubated at room temperature for 10 min to 15 min. 1 M H₂SO₄ was added at 50 µL/well to stop the reactions. The absorbance values at 450 nm were measured using a microplate reader. A curve for the inhibition of the binding of the ligand to the receptor was fitted using software, and IC₅₀ values were calculated. The ligand and receptor proteins used in the experiments are as follows: human RANKL protein (Sino Biological, 11682-HNCH), human RANK protein (Sino Biological, 16078-H02H), human TrkA protein (Sino Biological, 11073-H03H), and human NGF protein (Sino biological, 11050-HNAC).

The experimental results are shown in Table 9 below.

**Table 9. Blocking activity of bispecific antibody**

| Antibody | Blocking binding of RANKL to RANK | Blocking binding of NGF to TrkA |
|---|---|---|
| | IC₅₀ (nM) | IC₅₀ (nM) |
| Bispecific antibody 1 | 0.3225 | 102.1 |
| Denosumab | 0.5585 | / |
| Tanezumab | / | 3.663 |

The results show that the blocking activity of bispecific antibody 1 for RANKL is better than that of denosumab, and its blocking activity for NGF is significantly weaker than that of tanezumab. This indicates that reducing the binding activity of the NGF end of bispecific antibody 1 also significantly reduced its blocking activity for NGF.

### Test Example 6: Osteoclast Differentiation Experiments

The major function of RANKL is to promote the differentiation and maturation of osteoclasts. Therefore, the inhibitory activity of antibodies against RANKL can be tested by osteoclast differentiation experiments. The experimental procedures are as follows:
Raw264.7 cells (ECACC, 91062702) were plated in a 96-well cell culture plate (Corning, 3599) and incubated overnight in a 37 °C incubator. The next day, a fixed concentration of the human RANKL protein (Sino Biological, 11682-HNCH) and a serially diluted test bispecific antibody were added to the 96-well cell plate, and the plate was incubated at 37 °C for 4 days. The 96-well plate was taken out, and the supernatants were removed. A cell lysis buffer (Beyotime, P0013J) was added at 100 µL/well. After the mixtures were well mixed using a pipette, the cell lysates were transferred to centrifuge tubes, placed on ice for 10 min, and centrifuged, and the supernatants were collected. Referring to the method described in the tartaric acid-resistant acidic phosphatase assay kit (Beyotime, P0332), 40 µL of a test sample was taken and well mixed with 40 µL of a chromogenic substrate and 5 µL of tartaric acid; the mixture was incubated in a 37 °C incubator for 10 min, and 160 µL of stop solution was added; the OD values at 405 nm were measured using a microplate reader, fitting was performed on the data using software, and IC₅₀ values were obtained. The experimental results are shown in Table 10 below.

**Table 10. Osteoclast differentiation-inhibiting activity of bispecific antibody**

| Antibody | IC₅₀ (nM) |
|---|---|
| Bispecific antibody 1 | 1.193 |
| Denosumab | 3.917 |

The results show that the osteoclast differentiation-inhibiting activity of bispecific antibody 1 is more than 3.3 times that of denosumab.

### Test Example 7: TF-1 Cell Proliferation Experiment

NGF promotes the proliferation of TF-1 cells *in vitro.* Therefore, the inhibitory activity of antibodies against NGF was assessed by TF-1 cell proliferation experiments. The experimental procedures are as follows:
TF-1 cells (ATCC, CRL-2003) were resuspended in a GM-CSF-free 1640 medium (Gibco, 22400-105) and plated in a 96-well cell culture plate (Corning, 3903), and the plate was incubated in a 37 °C incubator overnight. The next day, a fixed concentration of the human NGF protein (Sino biological, 11050-HNAC) and a serially diluted test antibody were added to the 96-well cell plate, and the plate was incubated in a 37 °C incubator for 72 h. The cell culture plate was taken out, and Cell-titer Glo (Promega, G755B) solution was added at 50 µL/well. The plate was gently shaken for 10 min and left to stand at room temperature for 10 min, and bioluminescent signals were detected using PE Victor 3. Fitting was performed on the data using software, and IC₅₀ values were obtained. The experimental results are shown in Table 11 below.

**Table 11. TF-1 cell proliferation-inhibiting activity of bispecific antibody**

| Antibody | IC₅₀ (nM) |
|---|---|
| Bispecific antibody 1 | 3.159 |
| Tanezumab | 0.7589 |

The results show that bispecific antibody 1 can still inhibit TF-1 cell proliferation, and the inhibitory activity of tanezumab is about 4.2 times that of bispecific antibody 1, which indicates that the activity of the NGF end of bispecific antibody 1 was significantly reduced.

Therefore, on condition that there is a correlation between *in vitro* activity and dosage, we expect that the dosage of the RANKL end of bispecific antibody 1 can be reduced to about 40 mg, and the *in vivo* efficacy of the RANKL end can still be maintained; the dosage of the NGF end can be increased to about 80 mg, and the good safety can still be maintained. In addition, the clinical administration cycle of the antibody tanezumab against NGF is 2 times that of the antibody denosumab against RANKL at present. Therefore, the exemplary bispecific antibody 1 of the present disclosure can balance the dosages for the two targets.

### Test Example 8: In Vivo Efficacy in Animal Model of Bone Metastasis

The analgesic and bone-protecting effects of antibodies were assessed using an animal model of bone metastasis.

A construction method of the model: male C57 BL/6 mice, SPF, were purchased from Changzhou Cavens Laboratory Animal Co., Ltd. The animals were acclimatized to the laboratory environment, a 12/12-hour light/dark cycle was adopted, the temperature was 23±1 °C, and the humidity was 40% to 50%. The animals were given *ad libitum* access to standard sterile mouse feed and water. When the body weight of the mice reached about 25 g, modeling was started.

After 1% sodium barbiturate was intraperitoneally injected to anesthetize a mouse, the skin was prepared. The mouse was placed on a heating pad, a 1-cm cut was made, with ophthalmological scissors, in the skin on the lateral part of the knee of the left hind limb, in a direction parallel to the femur. The muscles were exposed, and the skin and muscle layers were isolated bluntly. An incision was made, with ophthalmological scissors, between rectus femoris and vastus medialis, along the lines in connective tissue. Rectus femoris and the patella were moved to the medial side of the knee joint with curved forceps. The femoral condyle was exposed without cutting off the patellar ligament. An opening was made, with a needle with a 0.45-mm diameter, from the top to the middle of the femur at the intercondylar fossa. The needle was inserted into the intramedullary space by 1 cm to 1.5 cm to form an injection channel. A Hamilton microsyringe (50 µL, model 1705 RN SYR, 26 gauge needle, model ga26/51 mm/pst3, catalog No. 7768-02) was inserted into the marrow cavity through the opening and 10 µL (5 × 10⁴) of LLC1 cells (ATCC, CRL-1642) was slowly injected. After the injection was complete, the syringe needle was pulled out. The leg was straightened, the patella and ligament were reset with curved forceps, and the muscles were restored to their original states. An antibiotic powder was sprinkled, and the outer skin cut was closed with an automatic wound clip (Roboz, Reflex 7 Clip 7 mm). Following the operation, animals were kept in cages of no more than three animals whenever possible. After seven days, wound clips were removed. The sham surgery group was subjected to the same operation, and an equal volume of PBS was injected into the marrow cavity. The blank group was normally kept without any treatment. Administration was started 7 days after the modeling and was performed once every 5 days and 3 times in total. Pain behaviors of mice were counted on day 14 (or day 15), and leg bone injury was scored on day 21.

Pain behavior counting method: Animals were placed on a wire mesh floor surrounded by organic glass and acclimatized for 30 min (until the animals stopped exploration and primary grooming activities in the box). Then their movements were observed, and behaviors of avoiding pain in the affected limb occurring within 5 min were assessed. The duration of the behaviors was measured with a stopwatch.

Behaviors of avoiding pain are defined as follows:
(1) complete protection (walking with the affected limb lifted, standing without it on the ground);
(2) curling the digits of the affected limb and sticking through the mesh (A normal mouse will have the digits of its limbs stretched and spread on the mesh and stand on the anterior parts of its paws. A tumor bone metastasis mouse will have the digits of its affected limb curled. If it does so and can stand on the heels, the pain is deemed to be mild, and the behavior does not count. If the entire soles of its paws are not in contact with the mesh or stick through the mesh, the behavior counts);
(3) licking limbs on the affected side;
(4) sporadic single-leg jumps; and
(5) standing on the hind limb of one side (with both forelimbs lifted).

Bone injury scoring method:
The left femur in the prone position was carefully dissected and examined for bone destruction using an MX-20 digital cabinet X-ray system (Faxitron/Bioptics), and bone injury was scored by a designated professional.

The scoring criteria are as follows:
0-relatively normal, 1-mild-local injury, 2-moderate-local injury, 3-moderate-multiple injuries, 4-severe-diffuse injury. Scoring was performed on the distal end of the femur and the proximal end of the femur separately, and the scores were added. Bone destruction scored 4 + 4 = 8 points when it was most severe.
1. Analgesic and bone-protecting effects of RANLK antagonists and NGF antagonists Since denosumab has no cross-binding activity for murine RANKL, we used the murine RANKL antibody AMR2 in combination with tanezumab to evaluate whether the combination of the two antibodies has better *in vivo* efficacy than one of them.

The variable region sequence of the antibody AMR2 is derived from WO2013176469A1. The variable region heavy and light chain sequences were fused to human IgG4 and human λ constant regions, respectively, to construct the full-length antibody AMR2. The related sequences of AMR2 are as follows:

**Table 12. CDRs of antibody AMR2**

| | | | |
|---|---|---|---|
| HCDR1 | DYDMS (SEQ ID NO: 32) | LCDR1 | TGSSSNIGNNAVS (SEQ ID NO: 35) |
| HCDR2 | WIYPSGGSIYYADSVKG (SEQ ID NO: 33) | LCDR2 | SDRHRPS (SEQ ID NO: 36) |
| HCDR3 | SGLTRTRWPIYYADGMDV (SEQ ID NO: 34) | LCDR3 | GSWDASLSGYV (SEQ ID NO: 37) |

AMR2 heavy chain variable region: 
EVQLLESGGGLVQPGGSLRLSCAASGFTFSDYDMSWVRQAPGKGLEWVSWIYP
SGGSIYYADSVKGRFTISRDNSKNTLYLQMNSLRAEDTAVYYCARSGLTRTRWPI
YYADGMDVWGQGTLVTVSS
SEQ ID NO: 38;
AMR2 light chain variable region: 
QSVLTQPPSASGTPGQRVTISCTGSSSNIGNNAVSWYQQLPGTAPKLLIYSDRHRP
SGVPDRFSGSKSGTSASLAISGLRSEDEADYYCGSWDASLSGYVFGGGTKLTVL
SEQ ID NO: 39;
AMR2 heavy chain 
EVQLLESGGGLVQPGGSLRLSCAASGFTFSDYDMSWVRQAPGKGLEWVSWIYP
SGGSIYYADSVKGRFTISRDNSKNTLYLQMNSLRAEDTAVYYCARSGLTRTRWPI
YYADGMDVWGQGTLVTVSSASTKGPSVFPLAPCSRSTSESTAALGCLVKDYFPE
PVTVSWNSGALTSGVHTFPAVLQSSGLYSLSSVVTVPSSSLGTKTYTCNVDHKPS
NTKVDKRVESKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQDWLNGK
EYKCKVSNKGLPSSIEKTISKAKGQPREPQVYTLPPSQEEMTKNQVSLTCLVKGF
 
YPSDIAVEWESNGQPENNYKTTPPVLDSDGSFFLYSRLTVDKSRWQEGNVFSCS
VMHEALHNHYTQKSLSLSLGK
SEQ ID NO: 40;
AMR2 light chain

QSVLTQPPSASGTPGQRVTISCTGSSSNIGNNAVSWYQQLPGTAPKLLIYSDRHRP
SGVPDRFSGSKSGTSASLAISGLRSEDEADYYCGSWDASLSGYVFGGGTKLTVL
GQPKANPTVTLFPPSSEELQANKATLVCLISDFYPGAVTVAWKADGSPVKAGVE
TTKPSKQSNNKYAASSYLSLTPEQWKSHRSYSCQVTHEGSTVEKTVAPTECS
SEQ ID NO: 41.

Grouping and dosing are shown in Table 13 below:

**Table 13. Grouping and dosing**

| Group | Number of mice | Antibody | Dose (mpk) | Frequency of administration | Route of administration |
|---|---|---|---|---|---|
| 1 | 12 | Vehicle | / | Q5d*3 | i.p. |
| 2 | 12 | Tanezumab | 10 | Q5d*3 | i.p. |
| 3 | 12 | AMR2 | 20 | Q5d*3 | i.p. |
| 4 | 12 | Tanezumab + AMR2 | 10 + 20 | Q5d*3 | i.p. |
| Sham | 10 | / | / | / | / |
| Blank | 10 | / | / | / | / |

| | | | | | |
|---|---|---|---|---|---|
| Notes: Q5d*3 indicates that the administration was performed once every 5 days and 3 times in total; i.p. indicates intraperitoneal administration. | | | | | |

The experimental results are shown in FIG. 2A to FIG. 2D.

The results of counting pain behaviors of mice show that on day 14, three administration groups showed significant analgesic effects compared to the negative group, and the analgesic effect of the combination administration group was stronger than those of the mAb groups. On day 21, only the combination administration group showed a significant analgesic effect. Although the two mAb groups showed certain analgesic effects, but their effects were not statistically different from that of the negative group (vs vehicle: *P < 0.05, **P < 0.01, ***P < 0.0001).

The results of bone injury scoring show that the combination administration group exhibited a certain bone-protecting effect on day 14. The combination administration group showed a significant bone-protecting effect on day 21. The above experimental results show that the combination administration group demonstrated efficacy in both pain relieving and bone protection. Thus, the inventors further verified the *in vivo* efficacy of the bispecific antibody molecules of the present disclosure.

### 2. Evaluation of in vivo efficacy of the NGF end of the bispecific antibody

As the *in vitro* activity of the NGF end of bispecific antibody 1 is about 4 times lower than the NGF mAb tanezumab, whether the NGF end of bispecific antibody 1 is still effective in mice was determined by this efficacy experiment. Grouping is shown in the table below:

**Table 14. Dosing and grouping**

| Group | Number of mice | Drug | Dose (mpk) | Frequency of administration | Route of administration |
|---|---|---|---|---|---|
| 1 | 12 | Vehicle | / | Q5d | i.p. |
| 2 | 12 | Bispecific antibody 1 | 13.5 | Q5d | i.p. |

| | | | | | |
|---|---|---|---|---|---|
| Notes: Q5d indicates that the administration was performed once every 5 days; i.p. indicates intraperitoneal administration. | | | | | |

The experimental results are shown in FIG. 3A to FIG. 3B.

The results show that bispecific antibody 1 exhibited a significant analgesic effect compared to the negative group on both day 14 and day 21 after administration, which indicates that the NGF end of bispecific antibody 1 is still significantly effective.

### 3. Evaluation of in vivo efficacy of the bispecific antibody

An animal model of bone metastasis was constructed using human RANKL transgenic mice (purchased from Biocytogen) to evaluate the *in vivo* efficacy of the bispecific antibody. Grouping is shown in Table 15 below:

**Table 15. Dosing and grouping**

| Group | Number of mice | Drug | Dose (mpk) | Frequency of administration | Route of administration |
|---|---|---|---|---|---|
| 1 | 10 | Sham | - | Q5d | i.p. |
| 2 | 10 | Vehicle | - | Q5d | i.p. |
| 3 | 14 | Bispecific antibody 1 | 5.4 | Q5d | i.p. |
| 4 | 14 | | 21.6 | Q5d | i.p. |

| | | | | | |
|---|---|---|---|---|---|
| Notes: Q5d indicates that the administration was performed once every 5 days; i.p. indicates intraperitoneal administration. | | | | | |

The experimental results are shown in FIG. 4A to FIG. 4D.

The results show that bispecific antibody 1 exhibited significant analgesic and bone-protecting effects. On both day 15 and day 21, the high-dose group of bispecific antibody 1 exhibited a significant analgesic effect, and both the high-dose and low-dose groups exhibited significant protective effects against bone injury.

### Test Example 9: PK Assays in Rats

An *in vivo* pharmacokinetic study was performed in SD rats. Male SD rats (Zhejiang Vital River Laboratory Animal Technology Co., Ltd.) were divided into groups of 4 and intravenously injected with bispecific antibody 1 at a dose of 4 mg/kg. Whole blood samples of 0.2 mL were collected from the administration groups before administration and 5 min, 8 h, 24 h, 48 h, 84 h, 9 d, 10 d, 14 d, 21 d, and 28 d after administration, and no anticoagulant was added. The blood samples were left at 4 °C for 30 min and centrifuged at 1000 g for 15 min. The serum in the upper layers was collected and placed into EP tubes and stored at -80 °C.

Serum concentration was determined by ELISA, and the pharmacokinetic parameters were calculated for the test antibody by Winnolin software. The results are shown below:

**Table 16. PK results for bispecific antibody 1**

| Test items | RANKL arm | NGF arm |
|---|---|---|
| t_{1/2} (day) | 15.09 | 14.30 |

The results show that bispecific antibody 1 pharmacokinetically performed well in rats; the half-lives of the RANKL and NGF ends are 15.09 days and 14.3 days, respectively, which are close.

### Preparation Examples-Anti-RANKL-NGF Bispecific Antibody Formulations

### Preparation Process for Exemplary Pharmaceutical Composition (Formulation) of Antibody

Step 1: the RANKL-NGF bispecific antibody and a stabilizer were prepared into a formulation stock solution containing this antibody; the formulation stock solution was sterilized and filtered by a 0.22 µm filter; and the filtrate was collected.

Step 2: the filling amount (the target filling amount was 1.0 mL/vial) was adjusted, vials were selected for filling, and samplings were performed at the beginning of the filling, during the filling, and at the end of the filling to detect the difference in filling amount. Step 3: The capping machine was started, aluminum caps were put on, and capping was carried out.

Step 4: Visual inspection was performed to confirm that the products have no defects, such as inaccurate filling amount and poor appearance. Carton labels were printed, cartons were folded, packing was performed, and the carton labels were put on the cartons.

### Appearance:

A visual method was used. The sample vial was wiped clean, and the color, clarity, and visible foreign matter of the sample were observed on a clarity tester under a white background and a black background at an illumination intensity of 1000-1500 lx.

Appearance detection instrument: Jingtuo Instrument YB-2A clarity tester.

### Melting temperature (Tm) and aggregation temperature (Tagg):

Tm is the temperature at which 50% of the components in a protein are denatured during the heating process; Tagg is the temperature at which the protein aggregates during the heating process. The sample was loaded into a Uni tube, and the analysis was run with a thermal ramp from 25 °C to 95 °C.

Instrument for Tm and Taag analysis: Uncle, instrument manufacturer: Unchained.

### SEC molecular exclusion chromatography:

This is a method for analyzing the separation of a solute by the relative relationship between the pore size of the gel pores and the size of the polymer sample molecule coil.

SEC% (SEC monomer content percentage) = A monomer/A total × 100% (A monomer represents the peak area of the main peak monomer in the sample, and A total represents the sum of all peak areas). ΔSEC% = SEC% of formulation after stability experiment - SEC% of formulation before stability experiment.
Instrument for SEC analysis: Agilent HPLC 1260.
Column: Tosoh, TSKgel G3000SWXL (7.8 mm × 30 cm, 5 µm).

### NR-CE capillary gel electrophoresis:

This is a method of moving the gel into a capillary as a supporting medium for electrophoresis and performing separation according to the molecular weight of the sample under a certain voltage.

NR-CE% (non-reduced CE purity percentage) = A main peak/A total × 100% (A main peak is the peak area of the main peak in the sample, and A total is the sum of all peak areas.) ΔNR-CE% = NR-CE% of formulation after stability experiment - NR-CE% of formulation before stability experiment.

Instrument for CE analysis: Beckman capillary electrophoresis apparatus, model: PA800 plus.

### icIEF imaged capillary isoelectric focusing electrophoresis:

The capillary is used as a separation channel, direct current voltage is applied to two ends of the capillary, an ampholyte solution in the capillary forms a certain range of pH gradient, and each component migrates to its own isoelectric point according to the difference in charge, focusing into a very narrow section, so as to achieve separation of the components.

icIEF% (main peak content percentage) = A main peak area/A total area × 100% (A total area represents the sum of areas of acidic, main, and basic peaks). ΔicIEF% = icIEF% of formulation after stability experiment - icIEF% of formulation before stability experiment.

Instrument for icIEF analysis: Protein Simple, model: Maurice.

The RANKL-NGF bispecific antibody used in the preparation examples of the formulations below was bispecific antibody 1 as described above, sometimes referred to simply as "antibody".

### Preparation Example 1. pH Screening for RANKL-NGF Bispecific Antibody Formulations

A 10 mM citric acid-disodium hydrogen phosphate buffer system was selected, 10 different pH values of 4.2, 4.6, 5.0, 5.4, 5.8, 6.2, 6.6, 7.0, 7.4, and 7.8 were designed, and 10 groups of formula antibody formulations with the concentration of the RANKL-NGF bispecific antibody being 20 mg/mL were prepared. The melting temperature (Tm) of the sample was measured to investigate the conformation stability of the antibody under different pH conditions, and the aggregation temperature (Tagg) of the sample was measured to investigate the colloidal stability of the antibody under different pH conditions. Also, the thermal stability of the antibody under different pH conditions was investigated by determining the appearance, SEC, NRCE, and the like of the sample at 40 °C.
1) 10 mM citric acid-disodium hydrogen phosphate, pH 4.2;
2) 10 mM citric acid-disodium hydrogen phosphate, pH 4.6;
3) 10 mM citric acid-disodium hydrogen phosphate, pH 5.0;
4) 10 mM citric acid-disodium hydrogen phosphate, pH 5.4;
5) 10 mM citric acid-disodium hydrogen phosphate, pH 5.8;
6) 10 mM citric acid-disodium hydrogen phosphate, pH 6.2;
7) 10 mM citric acid-disodium hydrogen phosphate, pH 6.6;
8) 10 mM citric acid-disodium hydrogen phosphate, pH 7.0;
9) 10 mM citric acid-disodium hydrogen phosphate, pH 7.4;
10) 10 mM citric acid-disodium hydrogen phosphate, pH 7.8.

**Table 17. pH screening result 1 for RANKL-NGF bispecific antibody formulations**

| No. | pH | Tm (°C) | Tagg (°C) |
|---|---|---|---|
| 1 | 4.2 | 66.06 | 63.58 |
| 2 | 4.6 | 69.10 | 64.81 |
| 3 | 5.0 | 69.60 | 64.97 |
| 4 | 5.4 | 68.81 | 64.96 |
| 5 | 5.8 | 68.34 | 64.12 |
| 6 | 6.2 | 67.26 | 63.39 |
| 7 | 6.6 | 66.06 | 62.78 |
| 8 | 7.0 | 65.06 | 62.45 |
| 9 | 7.4 | 63.39 | 62.58 |
| 10 | 7.8 | 60.21 | 62.09 |

**Table 18. pH screening result 2 for RANKL-NGF bispecific antibody formulations**

| No. | Appearance | | | |
|---|---|---|---|---|
| | Color | Clarity | Visible foreign matter | |
| | T0/40 °C | T0/40 °C | T0 | 40 °C-7 days |
| 1 | Colorless | Slightly opalescent | No visible particles or foreign matter | A small number of protein particles |
| 2 | Colorless | Slightly opalescent | No visible particles or foreign matter | A small number of protein particles |
| 3 | Colorless | Slightly opalescent | No visible particles or foreign matter | A small number of protein particles |
| 4 | Colorless | Slightly opalescent | No visible particles or foreign matter | A small number of protein particles |
| 5 | Colorless | Slightly opalescent | No visible particles or foreign matter | A large number of protein particles |
| 6 | Colorless | Slightly opalescent | No visible particles or foreign matter | A large number of protein particles |
| 7 | Colorless | Slightly opalescent | No visible particles or foreign matter | A large number of protein particles |
| 8 | Colorless | Slightly opalescent | No visible particles or foreign matter | A large number of protein particles |
| 9 | Colorless | Slightly opalescent | No visible particles or foreign matter | A large number of protein particles |
| 10 | Colorless | Slightly opalescent | No visible particles or foreign matter | A large number of protein particles |

**Table 19. pH screening result 3 for RANKL-NGF bispecific antibody formulations**

| No. | SEC-HPLC | | | | NRCE | | | |
|---|---|---|---|---|---|---|---|---|
| | Aggregate% | | Monomer% | | Main peak% | | Fragment% | |
| | T0 | 40 °C-7 days | T0 | 40 °C-7 days | T0 | 40 °C-7 days | T0 | 40 °C-7 days |
| 1 | 1.8 | 3.7 | 97.8 | 95.7 | 96.8 | 95.9 | 3.2 | 4.1 |
| 2 | 1.7 | 1.8 | 97.9 | 97.6 | 97.6 | 95.8 | 2.4 | 4.2 |
| 3 | 1.6 | 1.8 | 97.9 | 97.7 | 97.5 | 96.2 | 2.5 | 3.8 |
| 4 | 1.6 | 1.6 | 98.0 | 98.0 | 97.5 | 96.6 | 2.5 | 3.4 |
| 5 | 1.6 | 1.7 | 98.0 | 97.9 | 97.5 | 96.6 | 2.5 | 3.4 |
| 6 | 1.6 | 1.8 | 98.0 | 97.8 | 96.8 | 96.3 | 3.2 | 3.7 |
| 7 | 1.5 | 1.8 | 98.1 | 97.8 | 97.1 | 96.0 | 2.9 | 4.0 |
| 8 | 1.6 | 2.2 | 98.0 | 97.4 | 96.4 | 96.3 | 3.6 | 3.7 |
| 9 | 1.7 | 3.1 | 97.9 | 96.5 | 96.4 | 95.5 | 3.6 | 4.5 |
| 10 | 1.9 | 4.1 | 97.7 | 95.5 | 95.7 | 95.8 | 4.3 | 4.2 |

The results show that: the Tm values and Tagg values of the RANKL-NGF bispecific antibody were relatively high under the pH conditions of 4.6-5.4, which indicates that the antibody has relatively good conformation stability and colloidal stability in this pH range system; after investigation for 1 week at the high temperature (40 °C), the formulation exhibited a small number of visible protein particles in the pH range of 4.2-5.4, while a large number of protein particles were observed under other pH conditions. Purity: except that the proteins in the pH systems of 4.2, 7.4, and 7.8 were significantly aggregated after being incubated for 7 days at 40 °C and a small amount of aggregation was observed at pH 7.0, the proteins in the other pH systems were not significantly aggregated and had no significant difference among groups. NRCE results show that the fragment contents of the proteins in the pH systems of 4.6-5.8 were relatively low at T0, where the fragment proportions of the proteins in the pH of 5.0-5.8 after incubation at 40 °C for 7 days were lower than 4%. Combined with the molecular characteristics, appearance, and SEC results of the samples described above, it can be seen that the antibody performs best in the pH systems of 4.6-5.4, so that an intermediate pH value of 5.0 was selected as the optimal pH condition for subsequent research.

### Preparation Example 2. Buffer System Screening for RANKL-NGF Bispecific Antibody Formulations

Three groups of buffer systems, 10 mM acetic acid-sodium acetate with pH 4.8 and pH 5.2 and 10 mM histidine-histidine hydrochloride with pH 5.2, were selected, 75 mg/mL sucrose was used as a stabilizer, the concentration of the RANKL-NGF bispecific antibody was 20 mg/mL, and 3 groups of formula samples were prepared. By detecting the appearance, SEC-HPLC, NRCE, iCIEF purity, and the like of samples, 3 groups of formulas were investigated for stability under the conditions of storage at a high temperature (40 °C) for 2 weeks, storage at 25 °C for 2 weeks and 4 weeks, storage at 2-8 °C for 2 weeks and 4 weeks, repeated freeze-thaw cycles (-35 °C and room temperature) for 3 times and 5 times, and the like, so as to screen an optimal buffer system.
1) 10 mM acetic acid-sodium acetate, pH 4.8, 75 mg/mL sucrose, 20 mg/mL antibody;
2) 10 mM acetic acid-sodium acetate, pH 5.2, 75 mg/mL sucrose, 20 mg/mL antibody;
3) 10 mM histidine-histidine hydrochloride, pH 5.2, 75 mg/mL sucrose, 20 mg/mL antibody.

**Table 20. Buffer system screening result 1 for RANKL-NGF bispecific antibody formulations**

| No. | Appearance | | | | | | | | | | | | | | | | |
|---|---|---|---|---|---|---|---|---|---|---|---|---|---|---|---|---|---|
| | Color | Clarity | | | | | | | | Visible foreign matter | | | | | | | |
| | | T0 | 2~8 °C | | 25 °C | | 40 °C | Freeze-thaw | | T0 | 2~8 °C | | 25 °C | | 40 °C | Freeze-thaw | |
| | | | 2 weeks | 4 weeks | 2 weeks | 4 weeks | 2 weeks | 3 times | 5 times | | 2 week s | 4 weeks | 2 week s | 4 week s | 2 week s | 3 times | 5 times |
| 1 | Color less | Slight ly opale scent | Slightl y opales cent | Slightl y opales cent | Slightl y opalese ent | Slightl y opalese ent | Slightl y opalese ent | Slightl y opales cent | Slightl y opales cent | No visibl e partic les or foreig n matte r | A small numb er of fine partic les | A small numb er of fine particl es | A small numb er of fine partic les | A small numb er of fine partic les | A small numb er of fine partic les | A small numb er of fine partic les | A small numbe r of fine particl es |
| 2 | Color less | Slight ly opale scent | Slightl y opales cent | Slightl y opales cent | Opales cent | Opales cent | Opales cent | Slightl y opales cent | Slightl y opales cent | No visibl e partic les or foreig n matte r | A small numb er of fine partic les | A small numb er of fine particl es | A small numb er of fine partic les | A small numb er of fine partic les | A small numb er of fine partic les | A small numb er of fine partic les | A small numbe r of fine particl es |
| 3 | Color less | Slight ly opale scent | Slightl y opales cent | Slightl y opales cent | Slightl y opalese ent | Slightl y opalese ent | Slightl y opalese ent | Slightl y opales cent | Slightl y opales cent | No visibl e partic les or foreig n matte r | A small numb er of fine partic les | A small numb er of fine particl es | A small numb er of fine partic les | A small numb er of fine partic les | A large numb er of prote in partic les | A small numb er of fine partic les | A small numbe r of fine particl es |

**Table 21. Buffer system screening result 2 for RANKL-NGF bispecific antibody formulations**

| No. | SEC-HPLC | | | | | | | | | | | | | | | |
|---|---|---|---|---|---|---|---|---|---|---|---|---|---|---|---|---|
| | Aggregate% | | | | | | | | Monomer% | | | | | | | |
| | T0 | 2~8 °C | | 25 °C | | 40 °C | Freeze-thaw | | T0 | 2~8 °C | | 25 °C | | 40 °C | Freeze-thaw | |
| | | 2 weeks | 4 weeks | 2 weeks | 4 weeks | 2 weeks | 3 times | 5 times | | 2 weeks | 4 weeks | 2 weeks | 4 weeks | 2 weeks | 3 times | 5 times |
| 1 | 1.9 | 1.7 | 1.7 | 1.4 | 1.4 | 1.6 | 1.8 | 1.8 | 97.6 | 97.8 | 97.8 | 98.0 | 98.1 | 97.8 | 97.6 | 97.7 |
| 2 | 1.8 | 1.6 | 1.6 | 1.3 | 1.3 | 1.4 | 1.8 | 1.8 | 97.6 | 97.8 | 98.0 | 98.1 | 98.2 | 97.9 | 97.7 | 97.6 |
| 3 | 1.9 | 1.6 | 1.5 | 1.3 | 1.3 | 1.5 | 1.8 | 1.7 | 97.7 | 97.9 | 98.0 | 98.2 | 98.2 | 97.9 | 97.7 | 97.8 |

**Table 22. Buffer system screening result 3 for RANKL-NGF bispecific antibody formulations**

| No. | NRCE (Caliper) | | | | | | | | | | | | | | | |
|---|---|---|---|---|---|---|---|---|---|---|---|---|---|---|---|---|
| | Main peak% | | | | | | | | Fragment% | | | | | | | |
| | T0 | 2~8 °C | | 25 °C | | 40 °C | Freeze-thaw | | T0 | 2~8 °C | | 25 °C | | 40 °C | Freeze-thaw | |
| | | 2 weeks | 4 weeks | 2 weeks | 4 weeks | 2 weeks | 3 times | 5 times | | 2 week s | 4 week s | 2 week s | 4 week s | 2 weeks | 3 times | 5 times |
| 1 | 96.8 | 96.8 | 96.6 | 96.5 | 96.5 | 95.8 | 96.2 | 96.5 | 3.2 | 3.2 | 3.4 | 3.5 | 3.5 | 4.2 | 3.8 | 3.5 |
| 2 | 95.8 | 96.9 | 96.9 | 96.5 | 96.7 | 95.8 | 95.9 | 96.6 | 4.2 | 3.1 | 3.1 | 3.5 | 3.3 | 4.2 | 4.1 | 3.4 |
| 3 | 96.5 | 96.7 | 96.9 | 96.7 | 96.4 | 94.7 | 96.3 | 96.8 | 3.5 | 3.3 | 3.1 | 3.3 | 3.6 | 5.3 | 3.7 | 3.2 |

**Table 23. Buffer system screening result 4 for RANKL-NGF bispecific antibody formulations**

| N o. | iCIEF | | | | | | | | | | | | | | | | | | | | | | | |
|---|---|---|---|---|---|---|---|---|---|---|---|---|---|---|---|---|---|---|---|---|---|---|---|---|
| | Acidic peak% | | | | | | | | Main peak% | | | | | | | | Basic peak% | | | | | | | |
| | T0 | 2~8 °C | | 25 °C | | 40 °C | Freeze-th aw | | T0 | 2~8 °C | | 25 °C | | 40 °C | Freeze-th aw | | T0 | 2~8 °C | | 25 °C | | 40 °C | Freeze-th aw | |
| | | 2 wee ks | 4 wee ks | 2 wee ks | 4 wee ks | 2 wee ks | 3 time s | 5 time s | | 2 wee ks | 4 wee ks | 2 wee ks | 4 wee ks | 2 wee ks | 3 time s | 5 time s | | 2 wee ks | 4 wee ks | 2 wee ks | 4 wee ks | 2 wee ks | 3 time s | 5 time s |
| 1 | 34. 4 | 29.6 | 30.0 | 30.3 | 31.0 | 35.2 | 29.7 | 30.0 | 52. 2 | 57.3 | 57.2 | 58.0 | 58.8 | 51.0 | 56.9 | 56.7 | 13. 4 | 13.1 | 12.9 | 11.7 | 10.2 | 13.8 | 13.3 | 13.3 |
| 2 | 34. 0 | 29.7 | 29.1 | 30.5 | 31.2 | 35.9 | 30.2 | 29.9 | 52. 8 | 56.8 | 57.5 | 58.1 | 58.7 | 51.5 | 56.6 | 56.5 | 13. 1 | 13.5 | 13.3 | 11.4 | 10.1 | 12.6 | 13.3 | 13.7 |
| 3 | 33. 8 | 29.9 | 30.0 | 30.5 | 31.9 | 35.9 | 29.8 | 29.6 | 53. 2 | 56.7 | 56.9 | 57.7 | 57.6 | 50.3 | 56.7 | 56.9 | 13. 0 | 13.4 | 13.2 | 11.8 | 10.5 | 13.9 | 13.5 | 13.5 |

The results show that after incubation at 25 °C and 40 °C for 2 weeks, the protein in the system of 10 mM acetic acid-sodium acetate pH 5.2 was opalescent, the protein in the system of 10 mM acetic acid-sodium acetate pH 4.8 was slightly opalescent, and a small number of protein particles were observed in both formulas; after incubating the protein in the system of 10 mM histidine-histidine hydrochloride pH 5.2 at 40 °C for 2 weeks, a large number of protein particles were observed. Based on the appearance results, the stability of the protein in the 10 mM acetic acid-sodium acetate buffer system is better. Purity: the proportions of aggregates, monomers, and fragments of all groups of formulas under different stability conditions were not significantly changed compared with those of T0, and no difference existed among groups, which indicates relatively good SEC-HPLC purity stability; under the high-temperature condition, compared with the acetate buffer system, the NRCE fragment in the 10 mM histidine-histidine hydrochloride system was increased more significantly; the iCIEF detection results show that all the formulas had no significant difference. In conclusion, the 10 mM acetic acid-sodium acetate buffer system was selected for the subsequent formula development.

### Preparation Example 3. Concentration and Stabilizer Screening for RANKL-NGF Bispecific Antibody Formulations

To ensure a better pH buffering effect of the buffer system, the concentration of the buffer system was increased to 20 mM. 20 mM acetic acid-sodium acetate with pH 5.0 was selected as a buffer system, 0.2 mg/mL polysorbate 80 was selected as a surfactant, 75 mg/mL sucrose or 240 mM proline or 0.8% (w/v) sodium chloride was selected as a stabilizer, the antibody concentrations were 20 mg/mL, 50 mg/mL, and 70 mg/mL, respectively, and 5 groups of formula samples were prepared. The stability of the samples under shaking (300 rpm/25 °C), repeated freeze-thaw (-35 °C/room temperature), high temperature (40 °C), 25 °C, and illumination conditions was investigated.
1) 20 mM acetic acid-sodium acetate, pH 5.0, 75 mg/mL sucrose, 0.2 mg/mL polysorbate 80, and 50 mg/mL antibody;
2) 20 mM acetic acid-sodium acetate, pH 5.0, 240 mM proline, 0.2 mg/mL polysorbate 80, and 50 mg/mL antibody;
3) 20 mM acetic acid-sodium acetate, pH 5.0, 0.8% (w/v) sodium chloride, 0.2 mg/mL polysorbate 80, and 50 mg/mL antibody;
4) 20 mM acetic acid-sodium acetate, pH 5.0, 75 mg/mL sucrose, 0.2 mg/mL polysorbate 80, and 20 mg/mL antibody;
5) 20 mM acetic acid-sodium acetate, pH 5.0, 75 mg/mL sucrose, 0.2 mg/mL polysorbate 80, and 70 mg/mL antibody.

**Table 24. Concentration and stabilizer screening for RANKL-NGF bispecific antibody formulations-result 1**

| No. | Appearance | | | | | | | |
|---|---|---|---|---|---|---|---|---|
| | T0 | Shaking 3 days | Illumination 5 days | Freeze-thaw 3 times | Freeze-thaw 5 times | 25 °C-4 weeks | 40 °C-2 weeks | 40 °C-4 weeks |
| 1 | Colorless, slightly opalescent, no visible protein particles | | | | | | | |
| 2 | Colorless, slightly opalescent, no visible protein particles | | | | | | | |
| 3 | Colorless, opalescent, no visible protein particles | | | | | | | |
| 4 | Colorless, slightly opalescent, no visible protein particles | | | | | | | |
| 5 | Colorless, slightly opalescent, no visible protein particles | | | | | | | |

**Table 25. Concentration and stabilizer screening for RANKL-NGF bispecific antibody formulations-result 2**

| No. | SEC-HPLC | | | | | | | | | | | | | | | |
|---|---|---|---|---|---|---|---|---|---|---|---|---|---|---|---|---|
| | Aggregate% | | | | | | | | Monomer% | | | | | | | |
| | T0 | Shaking | Illumina tion | Freeze-thaw | | 25 °C | 40 °C | | T0 | Shaking | Illuminatio n | Freeze-thaw | | 25 °C | 40 °C | |
| | | 3 days | 5 days | 3 times | 5 times | 4 week s | 2 week s | 4 week s | | 3 days | 5 days | 3 times | 5 times | 4 week s | 2 week s | 4 week s |
| 1 | 1.5 | 1.4 | 1.6 | 1.5 | 1.5 | 1.3 | 1.7 | 2.2 | 98. 1 | 98.2 | 98.0 | 98.1 | 98.1 | 98.2 | 97.8 | 96.3 |
| 2 | 1.5 | 1.3 | 1.5 | 1.5 | 1.4 | 1.3 | 1.7 | 2.1 | 98. 1 | 98.2 | 98.0 | 98.1 | 98.1 | 98.2 | 97.7 | 96.4 |
| 3 | 1.5 | 1.5 | 1.8 | 1.5 | 1.7 | 1.6 | 3.9 | 4.5 | 98. 1 | 98.0 | 97.8 | 98.0 | 98.0 | 97.9 | 94.6 | 91.7 |
| 4 | 1.5 | 1.3 | 1.4 | 1.4 | 1.5 | 1.2 | 1.5 | 1.9 | 98. 1 | 98.2 | 98.1 | 98.1 | 98.0 | 98.2 | 98.0 | 96.6 |
| 5 | 1.4 | 1.4 | 1.9 | 1.5 | 1.4 | 1.4 | 1.9 | 2.6 | 98. 1 | 98.2 | 97.7 | 98.1 | 98.1 | 98.0 | 97.6 | 95.8 |

**Table 26. Concentration and stabilizer screening for RANKL-NGF bispecific antibody formulations-result 3**

| No. | NRCE | | | | | | | | | | | | | |
|---|---|---|---|---|---|---|---|---|---|---|---|---|---|---|
| | Main peak% | | | | | | | Fragment% | | | | | | |
| | T0 | Shakin g | Illuminati on | Freeze-tha w | 25 °C | 40 °C | | T0 | Shakin g | Illuminati on | Freeze-tha w | 25 °C | 40 °C | |
| | | 3 days | 5 days | 5 times | 4 weeks | 2 weeks | 4 weeks | | 3 days | 5 days | 5 times | 4 week s | 2 week s | 4 wee ks |
| 1 | 96.8 | 96.3 | 96.4 | 96.3 | 95.5 | 94.8 | 85.0 | 3.2 | 3.7 | 3.6 | 3.7 | 4.5 | 5.2 | 15.0 |
| 2 | 96.3 | 95.8 | 96.2 | 96.4 | 96.0 | 95.2 | 93.8 | 3.7 | 4.2 | 3.8 | 3.6 | 4.0 | 4.8 | 6.2 |
| 3 | 96.1 | 96.2 | 95.7 | 96.0 | 95.9 | 96.1 | 89.8 | 3.9 | 3.8 | 4.3 | 4.0 | 4.1 | 3.9 | 10.2 |
| 4 | 96.4 | 95.4 | 95.6 | 96.6 | 95.8 | 94.2 | 70.6 | 3.6 | 4.6 | 4.4 | 3.4 | 4.2 | 5.8 | 29.4 |
| 5 | 96.5 | 96.3 | 96.3 | 95.5 | 96.2 | 94.7 | 87.5 | 3.5 | 3.7 | 3.7 | 4.5 | 3.8 | 5.3 | 12.5 |

**Table 27. Concentration and stabilizer screening for RANKL-NGF bispecific antibody formulations-result 4**

| N o. | icIEF purity | | | | | | | | | | | | | | | | | | | | |
|---|---|---|---|---|---|---|---|---|---|---|---|---|---|---|---|---|---|---|---|---|---|
| | Acidic peak% | | | | | | | Main peak% | | | | | | | Basic peak% | | | | | | |
| | T 0 | Shak ing | Illumina tion | Freeze-t haw | 25 °C | 40 °C | | T 0 | Shak ing | Illumina tion | Freeze-t haw | 25 °C | 40 °C | | T 0 | Shak ing | Illumina tion | Freeze-t haw | 25 °C | 40 °C | |
| | | 3 days | 5 days | 5 times | 4 we eks | 2 we eks | 4 we eks | | 3 days | 5 days | 5 times | 4 we eks | 2 we eks | 4 we eks | | 3 days | 5 days | 5 times | 4 we eks | 2 we eks | 4 we eks |
| 1 | 36 .5 | 36.8 | 38.5 | 36.2 | 37. 4 | 41. 8 | 52. 0 | 49 .7 | 49.7 | 48.0 | 50.1 | 49. 5 | 43. 5 | 34. 7 | 13 .8 | 13.6 | 13.5 | 13.8 | 13. 1 | 14. 7 | 13. 3 |
| 2 | 36 .2 | 36.3 | 38.9 | 36.5 | 36. 8 | 40. 2 | 44. 9 | 50 .4 | 50.2 | 47.3 | 49.8 | 50. 1 | 45. 0 | 39. 4 | 13 .3 | 13.5 | 13.9 | 13.8 | 13. 1 | 14. 8 | 15. 7 |
| 3 | 36 .8 | 36.5 | 37.8 | 36.8 | 37. 9 | 40. 3 | 44. 0 | 50 .0 | 50.0 | 48.8 | 49.7 | 48. 3 | 43. 1 | 39. 0 | 13 .2 | 13.5 | 13.4 | 13.5 | 13. 8 | 16. 6 | 17. 0 |
| 4 | 37 .0 | 37.0 | 37.6 | 37.4 | 37. 6 | 40. 2 | 57. 6 | 49 .1 | 49.5 | 48.4 | 48.8 | 48. 7 | 45. 1 | 30. 5 | 13 .9 | 13.6 | 13.9 | 13.9 | 13. 7 | 14. 8 | 11. 9 |
| 5 | 37 .3 | 37.6 | 40.5 | 37.4 | 37. 7 | 40. 9 | 50. 2 | 49 .0 | 48.8 | 46.0 | 48.8 | 49. 8 | 44. 3 | 35. 6 | 13 .7 | 13.6 | 13.6 | 13.8 | 12. 5 | 14. 7 | 14. 2 |

The results show that compared with other formulas, formula 3 had slightly heavy opalescence and slightly poor appearance; in terms of purity, the contents of SEC-HPLC aggregates and NRCE fragments of all the formulas had no significant change under the shaking, illumination, repeated freeze-thaw, and 25 °C investigation conditions. Under the condition of high temperature of 40 °C, the content of the SEC-HPLC aggregates in formula 3 was significantly increased, which indicates that this formula has poor stability, and the content of the NRCE fragments in formula 2 was slightly increased, which indicates that proline can be used as a stabilizer to significantly improve the stability of the antibody, and the higher the concentration of the antibody is, the higher the NRCE purity is (the difference between the formulas of 50 mg/mL and 70 mg/mL was relatively small). Under the shaking, repeated freeze-thaw, and 25 °C investigation conditions, all the formulas had no significant change in iCIEF acidic, neutral, and basic peaks, but under the condition of high temperature of 40 °C for 4 weeks, the acidic peaks were all increased, the content of the main peak in formula 2 was highest, and the difference between the formulas of 50 mg/mL and 70 mg/mL was relatively small. Based on the above results, proline was selected as the stabilizer, the concentration of the antibody was 70 mg/mL, and the pH was 5.0.

### Preparation Example 4. Surfactant Concentration Screening for RANKL-NGF Bispecific Antibody Formulations

20 mM acetic acid-sodium acetate with pH 5.0 was selected as a buffer system, 240 mM proline was selected as a stabilizer, the concentration of the RANKL-NGF bispecific antibody was 70 mg/mL, and 3 groups of formula samples with concentrations of polysorbate 80 of 0.1 mg/mL, 0.2 mg/mL, and 0.4 mg/mL, respectively, were prepared. The stability of the samples under shaking (300 rpm/25 °C), repeated freeze-thaw (-35 °C/room temperature), and high temperature (40 °C) conditions was investigated.
1) 20 mM acetic acid-sodium acetate, pH 5.0, 240 mM proline, 0.1 mg/mL polysorbate 80, and 70 mg/mL antibody;
2) 20 mM acetic acid-sodium acetate, pH 5.0, 240 mM proline, 0.2 mg/mL polysorbate 80, and 70 mg/mL antibody;
3) 20 mM acetic acid-sodium acetate, pH 5.0, 240 mM proline, 0.4 mg/mL polysorbate 80, and 70 mg/mL antibody.

**Table 28. Surfactant concentration screening for RANKL-NGF bispecific antibody formulations-result 1**

| No. | Appearance | | | | | |
|---|---|---|---|---|---|---|
| | Color | Clarity | | | | Visible foreign matter |
| | All conditions | T0 | Shaking for 3 days/freeze-thaw for 3 times/freeze-thaw for 5 times | High temperature for 2 weeks | High temperature for 4 weeks | All conditions |
| 1 | Colorless | Clear and transparent | Clear and transparent | Slightly opalescent | Slightly opalescent | No visible protein particles |
| 2 | | Clear and transparent | Clear and transparent | Slightly opalescent | Slightly opalescent | |
| 3 | | Clear and transparent | Clear and transparent | Slightly heavy opalescence | Slightly heavy opalescence | |

**Table 29. Surfactant concentration screening for RANKL-NGF bispecific antibody formulations-result 2**

| No. | SEC-HPLC | | | | | | | | | | | |
|---|---|---|---|---|---|---|---|---|---|---|---|---|
| | Aggregate% | | | | | | Monomer% | | | | | |
| | T0 | Shaking for 3 days | Freeze-thaw | | High temperature | | T0 | Shaking for 3 days | Freeze-thaw | | High temperature | |
| | | | 3 times | 5 times | 2 weeks | 4 weeks | | | 3 times | 5 times | 2 weeks | 4 weeks |
| 1 | 2.2 | 1.2 | 1.8 | 1.7 | 1.7 | 2.2 | 97.6 | 98.7 | 98.1 | 98.2 | 97.9 | 97.1 |
| 2 | 2.2 | 1.2 | 1.8 | 1.7 | 1.7 | 2.3 | 97.6 | 98.7 | 98.1 | 98.3 | 97.9 | 97.0 |
| 3 | 2.2 | 1.3 | 1.8 | 1.7 | 1.8 | 2.5 | 97.6 | 98.6 | 98.1 | 98.3 | 97.8 | 96.9 |

**Table 30. Surfactant concentration screening for RANKL-NGF bispecific antibody formulations-result 3**

| Group | NRCE | | | | | |
|---|---|---|---|---|---|---|
| | Monomer% | | | Fragment% | | |
| | T0 | High temperature 2 weeks | High temperature 4 weeks | T0 | High temperature 2 weeks | High temperature 4 weeks |
| 1 | 98.1 | 96.9 | 95.8 | 1.9 | 3.1 | 4.2 |
| 2 | 98.1 | 96.8 | 90.7 | 1.9 | 3.2 | 9.3 |
| 3 | 97.8 | 94.3 | 86.3 | 2.2 | 5.7 | 13.7 |

**Table 31. Surfactant concentration screening for RANKL-NGF bispecific antibody formulations-result 4**

| Group | iCIEF | | | | | | | | |
|---|---|---|---|---|---|---|---|---|---|
| | Acidic peak% | | | Main peak% | | | Basic peak% | | |
| | T0 | High temperature 2 weeks | High temperature 4 weeks | T0 | High temperature 2 weeks | High temperature 4 weeks | T0 | High temperature 2 weeks | High temperature 4 weeks |
| 1 | 32.2 | 36.1 | 42.1 | 56.0 | 51.0 | 43.9 | 11.8 | 12.9 | 14.0 |
| 2 | 31.6 | 37.9 | 44.7 | 57.3 | 49.3 | 42.1 | 11.2 | 12.8 | 13.1 |
| 3 | 31.2 | 38.0 | 46.4 | 57.5 | 48.9 | 41.3 | 11.2 | 13.1 | 12.3 |

The result shows that the contents of the aggregate, the fragment, and the acidic and basic peaks tended to increase along with the increase of the concentration of polysorbate 80, and the stability of the antibody was relatively poor when the concentration of polysorbate 80 was 0.4 mg/mL. Therefore, the concentration of polysorbate 80 was preferably 0.1 mg/mL.

### Preparation Example 5. Stability Study on Preferred Formulas of RANKL-NGF Bispecific Antibody

A 20 mM acetic acid-sodium acetate buffer system with pH 5.0 was selected, 240 mM proline was used as a stabilizer, the concentration of the RANKL-NGF bispecific antibody was 70 mg/mL, and the formulation with the concentration of polysorbate 80 of 0.1 mg/mL was prepared. The stability of the formulation under an accelerated condition (25±2 °C/60% RH±5 RH) and a long-term condition (2-8 °C) was investigated.

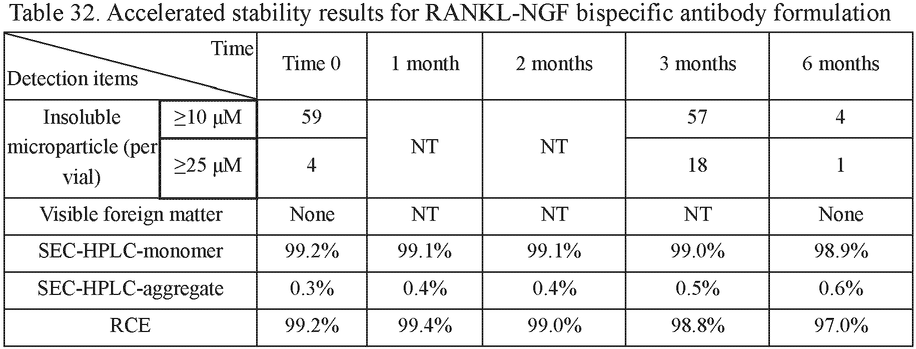

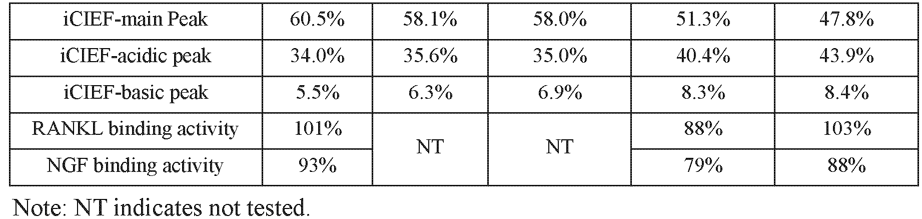

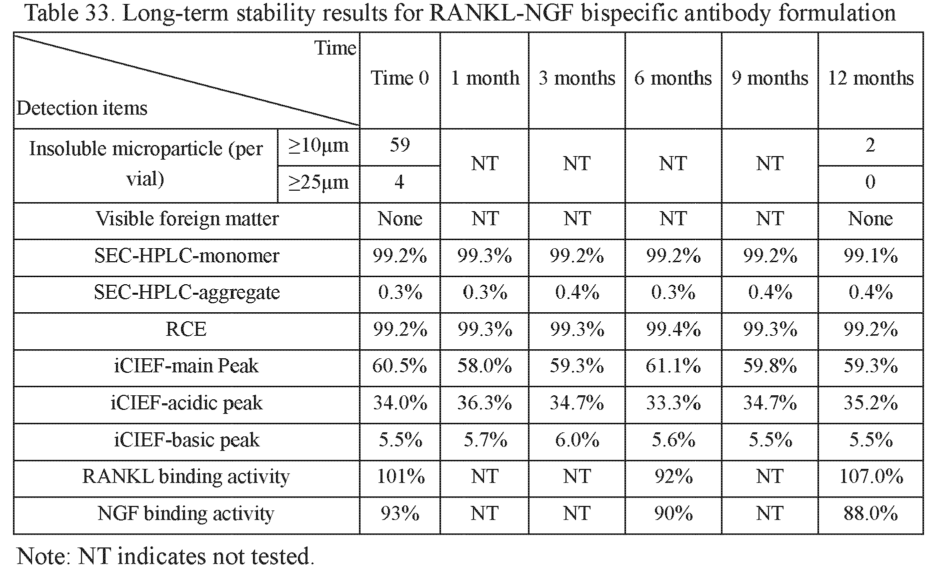

The results show that the main peak of iCIEF was reduced by about 12.7% when the sample was stored for 6 months under the accelerated condition (25±2 °C/60% RH±5 RH), and other detection items had no significant change; and when the sample was stored for 12 months under the long-term condition (2-8 °C), all detection items had no significant change compared with those of Time 0, indicating that the RANKL-NGF bispecific antibody formulation has good long-term stability.

### Preparation Example 6. Alternative Formulas

The present disclosure provides RANKL-NGF bispecific antibody pharmaceutical formulations of "50-77 mg/mL RANKL-NGF bispecific antibody, 240 mM proline, 0.1 mg/mL polysorbate 80, and 20 mM acetic acid-sodium acetate, pH 4.6-5.4" formulas, including but not limited to:
(1) 70 mg/mL RANKL-NGF bispecific antibody, 240 mM proline, 0.1 mg/mL polysorbate 80, and 20 mM acetic acid-sodium acetate, pH 4.6;
(2) 70 mg/mL RANKL-NGF bispecific antibody, 240 mM proline, 0.1 mg/mL polysorbate 80, and 20 mM acetic acid-sodium acetate, pH 5.0;
(3) 70 mg/mL RANKL-NGF bispecific antibody, 240 mM proline, 0.1 mg/mL polysorbate 80, and 20 mM acetic acid-sodium acetate, pH 5.4;
(4) 50 mg/mL RANKL-NGF bispecific antibody, 240 mM proline, 0.1 mg/mL polysorbate 80, and 20 mM acetic acid-sodium acetate, pH 4.6;
(5) 50 mg/mL RANKL-NGF bispecific antibody, 240 mM proline, 0.1 mg/mL polysorbate 80, and 20 mM acetic acid-sodium acetate, pH 5.0;
(6) 50 mg/mL RANKL-NGF bispecific antibody, 240 mM proline, 0.1 mg/mL polysorbate 80, and 20 mM acetic acid-sodium acetate, pH 5.4;
(7) 77 mg/mL RANKL-NGF bispecific antibody, 240 mM proline, 0.1 mg/mL polysorbate 80, and 20 mM acetic acid-sodium acetate, pH 4.6;
(8) 77 mg/mL RANKL-NGF bispecific antibody, 240 mM proline, 0.1 mg/mL polysorbate 80, and 20 mM acetic acid-sodium acetate, pH 5.0;
(9) 77 mg/mL RANKL-NGF bispecific antibody, 240 mM proline, 0.1 mg/mL polysorbate 80, and 20 mM acetic acid-sodium acetate, pH 5.4.

The experimental results show that RANKL-NGF bispecific antibody formulations of the above formulas have good stability; the above formulas can be applied to the preparation of RANKL-NGF bispecific antibody medicaments.

## Claims

1. A pharmaceutical composition, comprising an anti-RANKL-NGF bispecific antibody and a buffer, wherein:
the anti-RANKL-NGF bispecific antibody comprises a first antigen-binding domain that specifically binds to RANKL and a second antigen-binding domain that specifically binds to NGF;
the buffer is an acetate buffer, a histidine buffer, or a phosphate buffer;
preferably, the buffer is an acetic acid-sodium acetate buffer, a histidine-histidine hydrochloride buffer, or a citric acid-disodium hydrogen phosphate buffer;
more preferably, the buffer is an acetic acid-sodium acetate buffer or a histidine-histidine hydrochloride buffer;
most preferably, the buffer is an acetic acid-sodium acetate buffer.

2. The pharmaceutical composition according to claim 1, wherein the pharmaceutical composition has a pH of 4.2 to 7.0;
preferably, the pharmaceutical composition has a pH of 4.6 to 5.4;
more preferably, the pharmaceutical composition has a pH of 4.8 to 5.2.

3. The pharmaceutical composition according to claim 1 or 2, wherein the anti-RANKL-NGF bispecific antibody is at a concentration of 1 mg/mL to 150 mg/mL;
preferably, the anti-RANKL-NGF bispecific antibody is at a concentration of 10 mg/mL to 80 mg/mL;
more preferably, the anti-RANKL-NGF bispecific antibody is at a concentration of 63 mg/mL to 77 mg/mL.

4. The pharmaceutical composition according to any one of claims 1 to 3, wherein the pharmaceutical composition comprises a surfactant;
preferably, the surfactant is polysorbate or poloxamer;
more preferably, the surfactant is polysorbate 20 or polysorbate 80;
most preferably, the surfactant is polysorbate 80.

5. The pharmaceutical composition according to claim 4, wherein the surfactant is at a concentration of 0.01 mg/mL to 1.0 mg/mL;
preferably, the surfactant is at a concentration of 0.01 mg/mL to 0.6 mg/mL;
more preferably, the surfactant is at a concentration of 0.01 mg/mL to 0.2 mg/mL;
most preferably, the surfactant is at a concentration of 0.05 mg/mL to 0.15 mg/mL.

6. The pharmaceutical composition according to any one of claims 1 to 5, wherein the pharmaceutical composition comprises a stabilizer;
preferably, the stabilizer is selected from one or more of the group consisting of proline, sucrose, trehalose, sorbitol, arginine, glycine, and sodium chloride;
more preferably, the stabilizer is proline, sucrose, or sodium chloride;
most preferably, the stabilizer is proline.

7. The pharmaceutical composition according to claim 6, wherein the stabilizer is at a concentration of 1 mM to 300 mM;
preferably, the stabilizer is at a concentration of 25 mM to 290 mM;
more preferably, the stabilizer is at a concentration of 210 mM to 270 mM.

8. The pharmaceutical composition according to any one of claims 1 to 7, wherein the buffer is at a concentration of 5 mM to 100 mM;
preferably, the buffer is at a concentration of 10 mM to 50 mM;
more preferably, the buffer is at a concentration of 10 mM to 30 mM;
most preferably, the buffer is at a concentration of 16 mM to 24 mM.

9. The pharmaceutical composition according to any one of claims 1 to 8, wherein:
the anti-RANKL-NGF bispecific antibody comprises:
at least one first antigen-binding domain that specifically binds to RANKL, and
at least one second antigen-binding domain that specifically binds to NGF;
preferably,
the anti-RANKL-NGF bispecific antibody comprises:
two first antigen-binding domains that specifically bind to RANKL, and
two second antigen-binding domains that specifically bind to NGF;
more preferably, the anti-RANKL-NGF bispecific antibody has a structure as shown in FIG. 1.

10. The pharmaceutical composition according to claim 9, wherein in the anti-RANKL-NGF bispecific antibody, the first antigen-binding domain that specifically binds to RANKL comprises a heavy chain variable region and a light chain variable region, wherein:
the heavy chain variable region comprises: a HCDR1 comprising the amino acid sequence of SEQ ID NO: 1, a HCDR2 comprising the amino acid sequence of SEQ ID NO: 2, and a HCDR3 comprising the amino acid sequence of SEQ ID NO: 3; and
the light chain variable region comprises: a LCDR1 comprising the amino acid sequence of SEQ ID NO: 4, a LCDR2 comprising the amino acid sequence of SEQ ID NO: 5, and a LCDR3 comprising the amino acid sequence of SEQ ID NO: 6;
preferably,
in the anti-RANKL-NGF bispecific antibody, the first antigen-binding domain that specifically binds to RANKL comprises a heavy chain variable region and a light chain variable region, wherein the heavy chain variable region comprises the amino acid sequence of SEQ ID NO: 7; and/or the light chain variable region comprises the amino acid sequence of SEQ ID NO: 8.

11. The pharmaceutical composition according to claim 9 or 10, wherein in the anti-RANKL-NGF bispecific antibody, the second antigen-binding domain that specifically binds to NGF comprises a heavy chain variable region and a light chain variable region, wherein:
the heavy chain variable region comprises: a HCDR1 comprising the amino acid sequence of SEQ ID NO: 15, a HCDR2 comprising the amino acid sequence of SEQ ID NO: 16, and a HCDR3 comprising the amino acid sequence of SEQ ID NO: 17; and
the light chain variable region comprises: a LCDR1 comprising the amino acid sequence of SEQ ID NO: 18, a LCDR2 comprising the amino acid sequence of SEQ ID NO: 19, and a LCDR3 comprising the amino acid sequence of SEQ ID NO: 20;
preferably,
in the anti-RANKL-NGF bispecific antibody, the second antigen-binding domain that specifically binds to NGF comprises a heavy chain variable region and a light chain variable region, wherein:
the heavy chain variable region comprises the amino acid sequence of SEQ ID NO: 21;
and/or
the light chain variable region comprises the amino acid sequence of SEQ ID NO: 22;
more preferably,
in the anti-RANKL-NGF bispecific antibody, the second antigen-binding domain that specifically binds to NGF comprises a heavy chain and a light chain, wherein the heavy chain comprises the amino acid sequence of SEQ ID NO: 24, and the light chain comprises the amino acid sequence of SEQ ID NO: 25.

12. The pharmaceutical composition according to any one of claims 1 to 11, comprising the following components:
(a) 1 mg/mL to 150 mg/mL said anti-RANKL-NGF bispecific antibody,
(b) 0.01 mg/mL to 1.0 mg/mL surfactant,
(c) 1 mM to 300 mM stabilizer, and
(d) 5 mM to 100 mM buffer, the pharmaceutical composition having a pH of 4.2 to 7.0;
wherein preferably, the pharmaceutical composition comprises the following components:
(a) 10 mg/mL to 80 mg/mL said anti-RANKL-NGF bispecific antibody,
(b) 0.01 mg/mL to 0.6 mg/mL polysorbate 20 or polysorbate 80,
(c) 25 mM to 290 mM proline, and
(d) 10 mM to 50 mM acetate buffer, the pharmaceutical composition having a pH of 4.6 to 5.4;
more preferably, the pharmaceutical composition comprises the following components:
(a) 63 mg/mL to 77 mg/mL said anti-RANKL-NGF bispecific antibody,
(b) 0.01 mg/mL to 0.2 mg/mL polysorbate 80,
(c) 210 mM to 270 mM proline, and
(d) 10 mM to 30 mM acetic acid-sodium acetate buffer, the pharmaceutical composition having a pH of 4.8 to 5.2;
most preferably, the pharmaceutical composition comprises the following components:
(a) 63 mg/mL to 77 mg/mL said anti-RANKL-NGF bispecific antibody,
(b) 0.05 mg/mL to 0.15 mg/mL polysorbate 80,
(c) 210 mM to 270 mM proline, and
(d) 16 mM to 24 mM acetic acid-sodium acetate buffer, the pharmaceutical composition having a pH of 4.8 to 5.2.

13. A lyophilized formulation, wherein the lyophilized formulation is capable of forming the pharmaceutical composition according to any one of claims 1 to 12 after being reconstituted.

14. A method for preparing a lyophilized formulation, comprising the step of lyophilizing the pharmaceutical composition according to any one of claims 1 to 12.

15. A lyophilized formulation, wherein the formulation is obtained by the method according to claim 14.

16. The pharmaceutical composition according to any one of claims 1 to 12, wherein the pharmaceutical composition is a formulation for subcutaneous, intravenous, intraperitoneal, or intramuscular injection, preferably a formulation for subcutaneous injection.

17. A method for treating or preventing a disease, comprising administering to a subject a therapeutically effective amount of the pharmaceutical composition according to any one of claims 1 to 12 or the lyophilized formulation according to claim 13 or 15,
wherein
preferably, the disease is pain, joint stiffness, or bone loss;
more preferably, the pain is selected from the group consisting of osteoarticular pain, rheumatoid arthritis pain, gout, bone cancer pain, fracture pain, post-surgical pain, cancer pain, painful bladder syndrome, musculoskeletal pain, prostatitis, pelvic pain, interstitial cystitis, lower back pain, dysmenorrhea, pain associated with bone disease, trigeminal neuralgia, post-herpetic neuralgia, herpes zoster infection, sciatica, migraine, diabetic neuropathy, and pain associated with peripheral nerves;
wherein the bone loss is associated with at least one condition selected from the group consisting of osteoporosis, Paget's disease, osteomyelitis, hypercalcemia, osteopenia, osteoporosis, osteonecrosis, bone injury, bone resorption, osteogenesis imperfecta, inflammation, autoimmune disease, enteritis, rheumatoid arthritis, systemic lupus erythematosus, Crohn's disease, periodontal bone resorption, osteolytic metastasis, and cancer.

18. The method according to claim 17, wherein the cancer is selected from the group consisting of breast cancer, prostate cancer, thyroid cancer, renal cancer, lung cancer, esophageal cancer, rectal cancer, bladder cancer, cervical cancer, ovarian cancer, liver cancer, gastrointestinal cancer, melanoma, multiple myeloma, osteosarcoma, lymphoma, non-small cell lung cancer, bone tumor, and Hodgkin's disease.
